# FASCICULE DE BREVET EUROPEEN

(11) **EP 0 727 489 B1**
(45) Date de publication et mention de la délivrance du brevet: **10.02.2010**
(21) Numéro de dépôt: 96400301.6
(22) Date de dépôt: 14.02.1996
(51) Int. Cl.: C12N 15/53, C12N 9/02, C12P 33/02, C12N 1/15, C12Q 1/68

(54) **Séquence d'ADN codant pour une protéine d'A. thaliana ayant une activité delta-5,7 stérol, delta-7 réductase, protéine delta-7-Red, procédé de production, souches de levures transformées, applications**
Für ein A. thaliana Protein kodierende DNA-Sequenz mit Delta-5,7-sterol-delta-7-Reduktaseaktivität, das Protein, Verfahren zur Herstellung, transformierte Hefe-Stämme und Verwendungen
DNA sequence coding for an A. thaliana protein with delta-5,7-sterol-delta-7-reductase activity, the protein, process for the production, transformed yeast-strains and use

(30) Priorité: 15.02.1995 FR 9501723; 01.06.1995 FR 9506517
(43) Date de publication de la demande: 21.08.1996
(73) Titulaire: Aventis Pharma S.A., 92160 Antony (FR)
(72) Inventeur: Chenivesse, Xavier, F-94200 Ivry sur Seine (FR); Duport, Catherine, F-91440 Bures sur Yvette (FR); Lecain, Eric, F-94230 Cachan (FR); Pompon, Denis, F-91190 Gif sur Yvette (FR)

(56) Documents cités:
- BIOCHEM. BIOPHYS. RES. COMMUN. (1991), 181(1), 465-73 CODEN: BBRCA9;ISSN: 0006-291X, 1991, XP002003627 TATON, MARYSE ET AL: "Identification of delta-5,7-sterol-delta-7-reductase in higher plant microsomes"

## Description

La présente invention concerne une séquence d'ADN codant pour une protéine d'A. thaliana ayant une activité delta-5,7 stérol,delta-7 réductase, la protéine delta-7Red, leur procédé de production, les souches de levures transformées et leurs applications.

La delta-5,7 stérol,delta-7 réductase (E.C.1.3.1.21) est une enzyme microsomale dont la présence a été mise en évidence par son activité dans des homogénats de foie de rat (M. E. Dempsey et al., Methods Enzymol., 15, 501-514, 1969) ainsi que dans des préparations de plants de Zea mays (M. Taton et al., Biochem. Biophys. Res. Commun., 181, 465-473, 1991). Cette réductase est dépendante du NADPH et réduit in vitro le 7-déhydrocholestérol en cholestérol.

Les stérols sont les constituants principaux des membranes chez les eucaryotes mais présentent des différences de structure selon les espèces. Dans les cellules d'eucaryotes tels que les levures, le stérol principal est l'ergostérol qui contient une double insaturation en C-5 et C-7, une chaîne latérale ramifiée en C-24 et une insaturation en C-22 tandis que chez les mammifères le cholestérol est caractérisé par une insaturation en C-5 et une chaîne latérale saturée. Le sitostérol, le stigmastérol et le campestérol, qui représentent les stérols les plus communs chez les plantes, ont une chaîne latérale ramifiée mais saturée et, comme les stérols des vertébrés, n'ont pas une insaturation en C-7. L'enzyme responsable de cette différence de structure du noyau stérol est la delta-5,7 stérol,delta-7 réductase.

La delta-5,7 stérol,delta-7 réductase n'a jamais été purifiée à homogénéité et une purification partielle a été seulement décrite (M. E. Dempsey et al. ; M. Taton et al., déjà cité). La protéine n'a pas été caractérisée par ses propriétés physico-chimiques. Aucune information sur la séquence de la protéine et aucun anticorps dirigé contre celle-ci n'ont été décrits. Par ailleurs, une apparente déficience en 7-déhydrocholestérol réductase a été décrite chez l'homme associée au syndrome RSH/Smith-Lemli-Opitz (SLO) (J. M. Opitz et al., Am. J. Med. Genet., 50, 326-338, 1994).

Le clonage d'un ADNc codant pour la delta-5,7 stérol, delta-7 réductase, qui peut permettre d'identifier la séquence de la protéine correspondante ainsi que la caractérisation du gène humain ou la mise en évidence d'une déficience congénitale de celui-ci, n'est donc pas réalisable avec les méthodes connues qui font l'usage par exemple des techniques d'hybridation ou/et de détection immunologique. L'obtention de méthodes de criblage inventives permettant de réaliser le clonage, notamment en absence d'informations sur la protéine, est donc particulièrement recherchée.

L'ergostérol, principal stérol des membranes des champignons, contient une paire de double liaisons conjuguées en C-5,7 qui confère une activité antimycotique aux composés de la famille des polyènes tel que la nystatine (R. Bittman et al., J. Biol. Chem., 260, 2884-2889, 1985). La forte dépendance de l'action de la nystatine à la concentration membranaire en stérols insaturés en C-5,7 a permis de sélectionner chez S. cerevisiae des souches de mutants vis-à-vis des stérols accumulés (S. W. Molzahn et al., J. Gen. Microbiol., 72, 339-348, 1972). Ainsi les mutants erg2 et erg3 accumulent des stérols qui n'ont pas les doubles liaisons conjuguées en C-5,7 en raison d'une déficience en stérol delta-8,7 isomérase (W. Ashman et al., Lipids, 26, 628, 1991) et en stérol delta-5 deshydrogénase (B. Arthinton et al., Gene, 102, 39, 1991) respectivement. De tels mutants sont viables parce que l'ergostérol, le stérol naturel principal de la levure, peut être remplacé dans certaines conditions par différents stérols de substitution incluant le cholestérol.

L'avantage de l'accroissement de la résistance à la nystatine de souches de levure enrichies en stérols n'ayant pas la double insaturation en C-5,7 a été perçu par les inventeurs pour cloner un ADNc codant une protéine hétérologue ayant une activité delta-5,7 stérol,delta-7 réductase avec une méthode de criblage utilisant une interférence métabolique dans S. cerevisiae. Le succès de cette approche qui offre l'avantage d'être indépendante de la connaissance de séquences de l'ADN ou de la protéine ainsi que d'une détection seulement basée sur l'activité enzymatique, n'est cependant pas prévisible en raison de nombreuses difficultés techniques à surmonter.

Une première limitation vient du fait que le mode d'action de la nystatine n'est pas entièrement élucidé (L. W. Parks et al., CRC Critical Reviews in Microbiology, 301-304, 1978). Par exemple, la faible spécificité de la sélection par la nystatine est prévisible en raison de la nature indirecte de celle-ci conduisant chez la levure à la sélection de mutations génomiques spontanées telles que les mutants erg (S. W. Molzahn et al. déjà cité) ou des mutations génomiques entraînant des résistances indépendantes de la voie des stérols. De même, les cellules transformées par une banque peuvent exprimer des gènes hétérologues conférant une résistance à la nystatine sans relation avec la voie des stérols.

Un autre exemple de limitation attendue concerne le fait que la protéine hétérologue puisse être faiblement active dans la cellule, conduisant à l'absence ou à une faible résistance à la nystatine par exemple pour l'une des raisons suivantes : 1) le gène codant pour la delta-5,7 stérol,delta-7 réductase est faiblement exprimé ; 2) la protéine est peu ou pas active parce que mal repliée ou résultant d'un adressage subcellulaire incorrect ; 3) la protéine de plante ne reconnait pas les stérols propres de la levure comme substrats ou 4) les stérols qui peuvent être des substrats sont sous forme estérifiée ou stockés dans des vésicules et ne sont pas au contact de l'enzyme. Il peut donc être prévu que les stérols ainsi accumulés échappent à l'action de la delta-5,7 stérol,delta-7 réductase qui, dans les eucaryotes, est réputée localisée dans les microsomes.

La présente invention concerne le clonage d'un ADNc d'A. thaliana codant pour une protéine ayant une activité delta-5,7 stérol,delta-7 réductase, désignée delta-7Red, selon un procédé de clonage réalisé dans une levure par interférence métabolique basée sur la résistance à la nystatine. La protéine delta-7Red permet de réduire des stérols ayant une insaturation en C-7 par un procédé de réduction biologique qui fournit de plus une solution avantageuse au problème de la réduction sélective en C-7 de stérols ou de stéroïdes ayant une double insaturation en C-5,7 que les méthodes de réduction par voie chimique ne permettent pas.

L'invention concerne également des cellules de levure transformées exprimant la delta-7Red qui, de façon surprenante, accumulent des produits saturés en C-7 et éventuellement saturés en C-22 qui, contrairement à l'ergostérol, sont des substrats de l'enzyme de clivage de la chaîne latérale du cholestérol, c'est-à-dire le cytochrome P₄₅₀SCC.

Ces propriétés inattendues permettent une utilisation des levures transformées de l'invention dans un procédé de préparation de stérols ou de stéroïdes ayant un intérêt industriel et/ou pharmacologique, en particulier la préparation de pregnénolone par oxydation biologique des stérols endogènes de levure, réduits en C-7, par le cytochrome P₄₅₀ SCC (P₄₅₀SCC) en présence d'adrénodoxine réductase (ADR) et d'adrénodoxine (ADX). Les levures transformées de l'invention peuvent être aussi utilisées dans un procédé de préparation des stéroïdes intermédiaires de la voie de métabolisation du cholestérol en hydrocortisone chez les mammifères et autres vertébrés. Cette utilisation présente l'avantage de permettre la préparation de l'hydrocortisone ou de ses intermédiaires dans un procédé d'oxydation biologique ne nécessitant pas l'emploi d'un stérol exogène tel que le cholestérol comme substrat initial et de contourner ainsi le problème de la pénétration d'un stérol dans la levure dont l'imperméabilité aux stérols exogènes sous des conditions d'aérobiose a été décrite (R. T. Lorentz et al., J. Bacteriology, 981-985, 1986).

L'invention concerne aussi l'utilisation des séquences nucléiques obtenues à l'aide du procédé de clonage de l'invention. Une séquence ARN ou ADN codant pour la delta-5,7 stérol,delta-7 réductase peut être utilisée pour le diagnostic ou le traitement d'affections impliquant le produit du gène de la delta-5,7 stérol,delta-7 réductase. Par exemple, une déficience en delta-5,7 stérol,delta-7 réductase qui convertit le 7-déhydro cholestérol en cholestérol est présumée impliquée dans le syndrome RSH/SLO. Ainsi une séquence d'ADN humain peut être utilisée comme sonde pour diagnostiquer une déficience en delta-5,7 stérol,delta-7 réductase et peut être aussi utilisée en thérapie génique pour corriger une telle déficience.

La présente invention a donc pour objet une séquence d'acide nucléique contenant une séquence codant pour une protéine ayant une activité delta-5,7 stérol,delta-7 réductase, ledit acide nucléique étant un ADN ou un ARN et particulièrement étant un ADNc.

L'activité delta-5,7 stérol,delta-7 réductase peut être mise en évidence par exemple à l'aide d'un test enzymatique in vitro décrit plus loin dans la partie expérimentale.

L'invention a plus particulièrement pour objet une séquence d'ADNc dans laquelle la séquence codante code pour une protéine d'A. thaliana ayant une activité delta-5,7 stérol,delta-7 réductase et ayant la séquence nucléotidique de la séquence SEQ ID N° 1 :

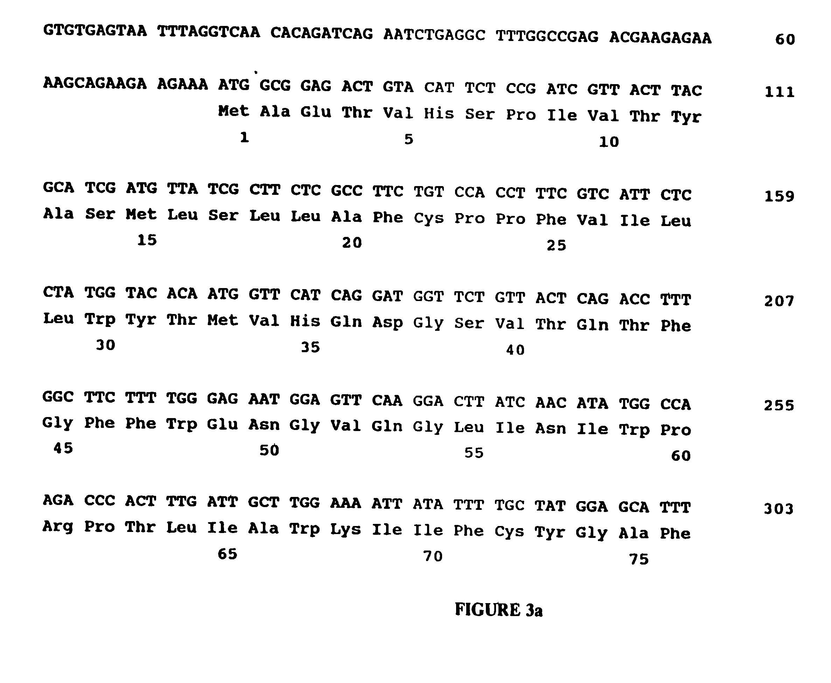

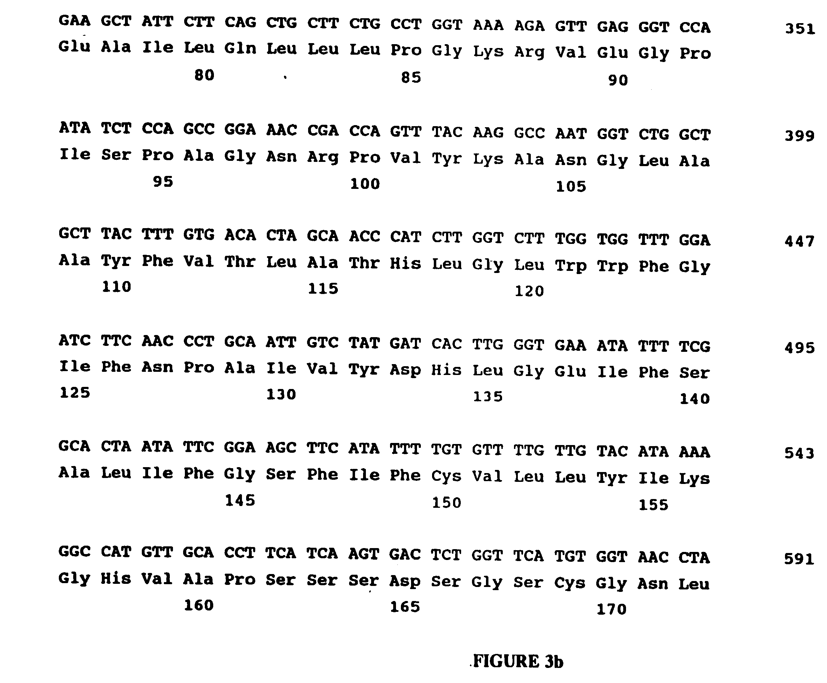

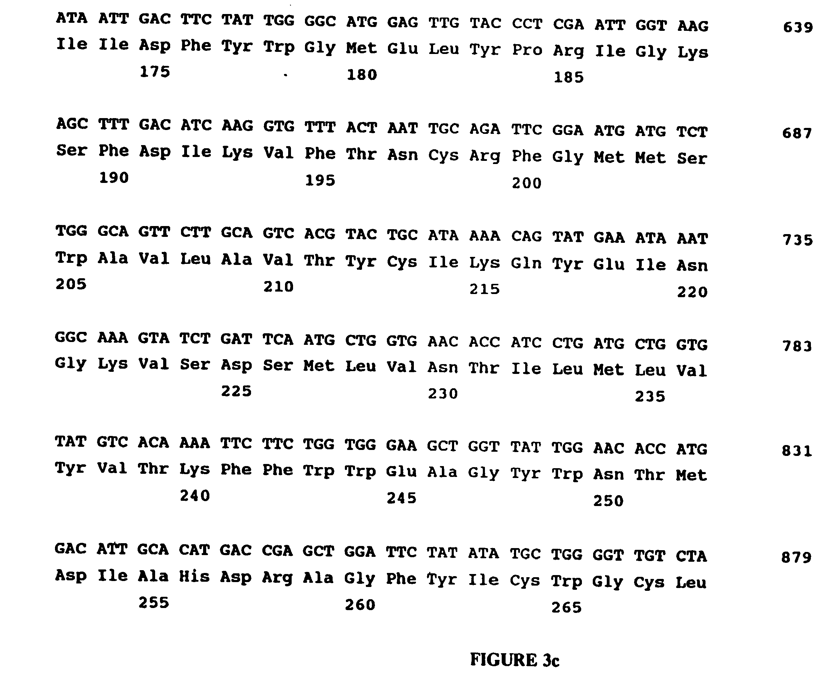

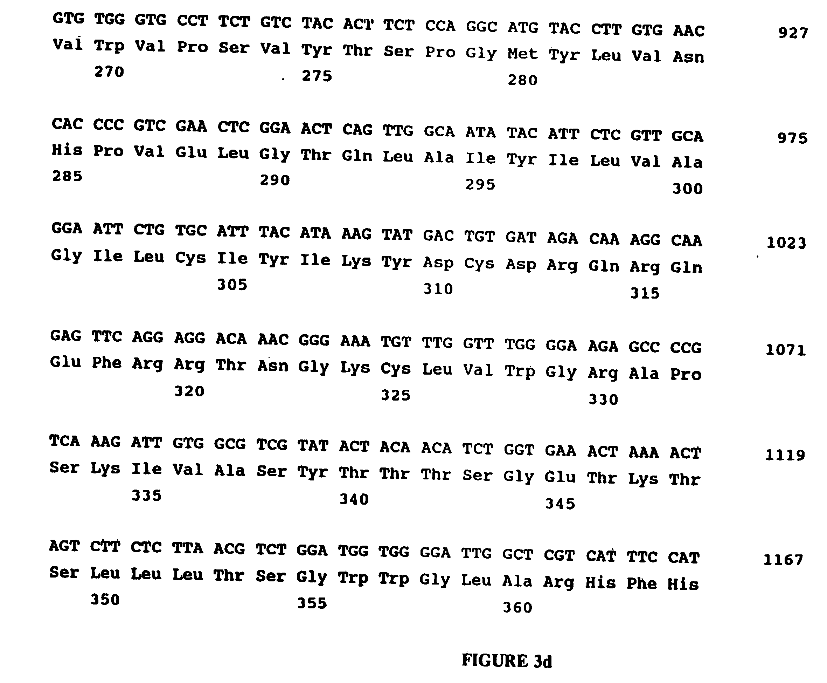

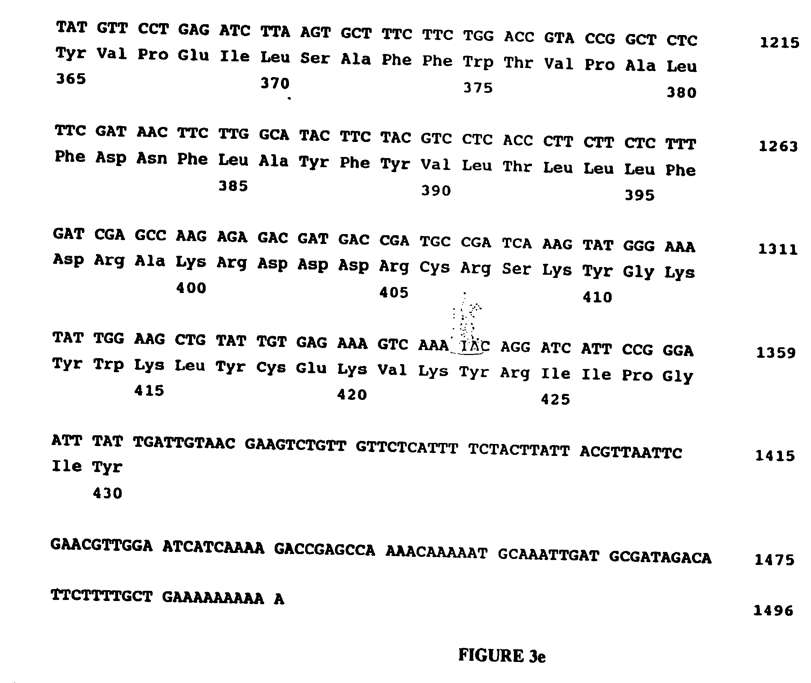

La séquence d'ADNc ci-dessus qui code pour une protéine ayant 430 acides aminés correspondant à la séquence montrée à la figure 3 (SEQ ID N° 2) est une séquence d'ADNc qui peut être obtenue par exemple par clonage dans une levure, à partir d'une banque d'expression d'A. thaliana, en utilisant une méthode de criblage fondée sur l'apparition d'une résistance de la levure à la nystatine, selon des conditions opératoires dont une description détaillée est donnée plus loin.

La connaissance de la séquence nucléotidique SEQ ID N° 1 ci-dessus permet de reproduire la présente invention par des méthodes connues de l'homme du métier, par exemple par synthèse chimique ou par criblage d'une banque génomique ou d'une banque d'ADNc à l'aide de sondes d'oligonucléotides de synthèse par les techniques d'hybridation ou par amplification par PCR.

L'invention a aussi pour objet une séquence d'ADN codant pour une protéine ayant une activité delta-5,7 stérol, delta-7 réductase et qui hybride à la séquence nucléotidique SEQ ID N° 1 dans des conditions de stringence moyenne ou élevée ou qui présente une identité de séquence d'environ 60 % et plus avec cette séquence.

Les séquences qui hybrident de façon détectable à la séquence SEQ ID N° 1 hybrident dans des conditions standards de stringence moyenne, par exemple une hybridation à 42°C, pendant 12 heures dans une solution 50 % formamide, SSCX6 suivie de lavages ou de stringence moins élevée, par exemple une hybridation à 42°C pendant 24 heures dans une solution 20 % formamide, SSCX6 suivie de lavages dans les conditions standards connues (T. Maniatis et al., Molecular cloning, Cold Spring Harbor Laboratory Press, 1989).

Le pourcentage d'identité de séquence nucléotidique peut être déterminé en utilisant par exemple le programme BLAST "basic local alignment search tool" (S. F. Altschul et al., J. Mol. Biol., 215, 403-410, 1990) sur le serveur NCBI.

L'invention concerne aussi une séquence d'ADN codant pour une protéine ayant une activité delta-5,7 stérol,delta-7 réductase et amplifiable par la technique de PCR en utilisant comme amorces des oligonucléotides codant pour une séquence consensus ayant la séquence en acides aminés SEQ ID N° 3 : dans laquelle Xaa en position 7 est Trp ou Tyr et Xaa en position 12 est His ou Lys associés à une amorce oligo dT.

La séquence SEQ ID N° 3 ci-dessus correspond à une séquence consensus nouvelle qui a été définie par alignement d'identité de séquences en acides aminés entre la séquence nouvelle SEQ ID N° 2, déduite de la séquence nucléotidique SEQ ID N° 1, et des séquences connues soit d'autres stérol réductases ayant une spécificité d'action à une position autre que la position C-7, soit de récepteurs à la lamin B, comme cela est détaillé plus loin dans la partie expérimentale. A partir de l'information donnée par la séquence en acides aminés SEQ ID N° 3, une amorce constituée d'au plus 45 nucléotides peut être définie et synthétisée qui, associée à une deuxième amorce oligodT (17 nucléotides) comme séquence de rebond, permettra à l'aide d'une trousse PCR du commerce (par exemple Stratagène) d'amplifier un ADN codant pour une protéine ayant l'activité delta-5,7 stérol,delta-7 réductase.

L'invention concerne aussi une protéine d'A. thaliana ayant une activité delta-5,7 stérol,delta-7 réductase et ayant la séquence en acides aminés SEQ ID N° 2 :

L'invention a spécialement pour objet une protéine d'A. thaliana ayant une activité delta-5,7 stérol,delta-7 réductase et ayant la séquence en acides aminés SEQ ID N° 2 ci-dessus et désignée delta-7Red.

L'invention concerne aussi une protéine ayant une activité délta-5,7 stérol,delta-7 réductase ayant une séquence en acides aminés présentant une identité de séquence d'environ 60 % et plus avec la séquence SEQ ID N° 2.

Le pourcentage d'identité peut être déterminé par exemple en utilisant le programme BLAST indiqué ci-dessus.

L'invention concerne également une protéine ayant une activité delta-5,7 stérol,delta-7 réductase et ayant une réactivité immunologique croisée avec la protéine d'A. thaliana delta-7Red définie ci-dessus.

La protéine peut être détectée par exemple par immuno-précipitation à l'aide d'un anti-sérum dirigé contre la protéine delta-7Red, préparé selon des méthodes connues.

Un des aspects de l'invention concerne une protéine ayant une activité delta 5,-7 stérol,delta-7 réductase telle qu'obtenue par expression dans une cellule hôte contenant une séquence d'ADN définie précédemment et concerne spécialement une protéine d'A. thaliana telle qu'obtenue par expression dans une cellule hôte contenant une séquence d'ADN codant pour la séquence en acides aminés SEQ ID N° 2 ci-dessus.

Lorsque la protéine de l'invention est obtenue par expression dans une cellule hôte, celle-ci est réalisée selon les méthodes de génie génétique et de culture cellulaire connues de l'homme du métier.

L'expression peut être réalisée dans une cellule hôte procaryote, par exemple E. coli ou dans une cellule hôte eucaryote, par exemple une cellule de mammifère, une cellule d'insecte ou une levure contenant la séquence codant pour la delta-7 Red de l'invention précédée d'un promoteur convenable.

La protéine recombinante obtenue peut être glycosylée ou non glycosylée.

L'invention concerne notamment une protéine selon l'invention telle qu'obtenue par expression dans une levure.

L'invention concerne aussi un anticorps dirigé contre une protéine ayant une activité delta-5,7 stérol,delta-7 réductase définie précédemment. L'anticorps peut être un anticorps polyclonal ou un anticorps monoclonal préparé selon les méthodes connues de l'homme du métier.

L'invention concerne aussi un vecteur d'expression contenant une séquence d'ADN définie précédemment ainsi qu'une cellule hôte transformée par ce vecteur.

Les vecteurs d'expression sont des vecteurs connus permettant l'expression de la protéine sous le contrôle d'un promoteur convenable. Pour les cellules procaryotes, le promoteur peut être par exemple le promoteur lac, le promoteur trp, le promoteur tac, le promoteur ß-lactamase ou le promoteur PL. Pour les cellules de mammifères, le promoteur peut être le promoteur SV40 ou les promoteurs de l'adéno-virus. Des vecteurs type Baculovirus peuvent être aussi utilisés pour l'expression dans des cellules d'insectes. Pour les cellules de levure, le promoteur peut être par exemple le promoteur PGK, le promoteur ADH, le promoteur CYC1 ou le promoteur GAL10/CYC1.

Les cellules hôtes peuvent être des cellules procaryotes ou des cellules eucaryotes. Les cellules procaryotes sont par exemple E. coli, Bacillus ou Streptomyces. Les cellules hôtes eucaryotes comprennent des levures et des champignons filamenteux ainsi que des cellules d'organismes supérieurs, par exemple des cellules de mammifères ou des cellules d'insectes. Les cellules de mammifères peuvent être des cellules CHO de hamster ou des cellules Cos de singe. Les cellules d'insectes sont par exemple des cellules SF9. Les cellules de levure peuvent être par exemple Saccharomyces cerevisiae, Schizosaccharomyces pombe ou Kluyveromyces lactis.

L'invention a aussi pour objet un procédé de clonage d'un acide nucléique codant pour une protéine ayant une activité delta-5,7 stérol,delta-7 réductase dans un micro-organisme comprenant une méthode de criblage choisie parmi
- la résistance du microorganisme à la nystatine ou à un composé analogue dont la toxicité dépend de la présence de stérols portant une insaturation en position C-7,
- l'hybridation de l'acide nucléique avec la séquence nucléotidique de la séquence SEQ ID N° 1 ci-dessus,
- l'identification de l'acide nucléique par voie informatique selon le progamme BLAST parmi des séquences d'ADN isolées au hasard.

Par microorganisme, on entend une levure telle que par exemple S. cerevisiae, S. pombe ou K. lactis.

Les composé analogues à la nystatine comprennent par exemple l'amphotéricine B ou la filipine.

Par hybridation, on entend une hybridation dans des conditions de stringence moyenne ou élevée selon les conditions standards connues (T. Maniatis et al., déjà cité).

L'identification par voie informatique est réalisée selon le programme BLAST indiqué ci-dessus.

Un exemple de procédé de clonage ci-dessus dans lequel la méthode de criblage utilise la résistance à la nystatine dans une levure est décrit plus loin dans la partie expérimentale.

L'invention a spécialement pour objet une cellule hôte, choisie parmi une levure ou un champignon filamenteux, transformée par un vecteur contenant une séquence d'ADN de l'invention définie précédemment.

L'un des objets de l'invention concerne aussi un procédé de préparation d'une protéine ayant une activité delta-5,7 stérol,delta-7 réductase dans lequel on cultive une cellule hôte transformée de l'invention et on isole la protéine exprimée et concerne particulièrement un procédé dans lequel la cellule hôte est une levure transformée dans laquelle la séquence d'ADN codante est placée sous le contrôle d'un promoteur de levure.

L'un des objets de l'invention concerne aussi un procédé de réduction in vitro d'un stérol insaturé en position C-7 dans lequel on incube le stérol à réduire avec la protéine obtenue par le procédé ci-dessus et on isole éventuellement le stérol réduit obtenu.

L'un des objets de l'invention concerne aussi un procédé de réduction in vivo d'un stérol exogène insaturé en position C-7 dans lequel on incube le stérol avec une cellule hôte transformée de l'invention et on isole éventuellement le stérol réduit obtenu. La cellule hôte peut être une cellule procaryote ou une cellule eucaryote, en particulier une levure ou un champignon filamenteux.

L'un des objets de l'invention concerne aussi un procédé de réduction in vivo d'un stérol endogène insaturé en position C-7 dans lequel on cultive une souche hôte transformée de l'invention choisie parmi une levure ou un champignon filamenteux et on isole éventuellement le stérol réduit accumulé.

L'invention concerne particulièrement un procédé de réduction in vitro ou in vivo défini ci-dessus dans lequel le stérol réduit obtenu est un substrat de l'enzyme de clivage de la chaîne latérale du cholestérol (P₄₅₀SCC) et concerne tout particulièrement un procédé de réduction in vivo dans lequel le stérol endogène à réduire est l'ergosta 5,7 diène 3-ol, l'ergosta 5,7,24(28) triène 3-ol ou l'ergosta 5,7,22 triène 3-ol ou un mélange de ceux-ci.

L'invention a aussi pour objet un procédé de production de pregnénolone dans lequel on cultive une cellule hôte transformée de l'invention choisie parmi une levure ou un champignon filamenteux, on isole éventuellement le ou les stérols endogènes réduits en position C-7 accumulés, on incube les stérols réduits en présence de P₄₅₀SCC, et éventuellement en présence d'adrénodoxine réductase (ADR) et d'adrénodoxine (ADX), et on isole éventuellement la pregnénolone obtenue.

L'invention a particulièrement pour objet un procédé de production de la pregnénolone défini ci-dessus dans lequel la cellule hôte est une levure.

L'invention concerne aussi un procédé de production de pregnénolone dans lequel on cultive une levure transformée par un ou plusieurs vecteurs permettant la coexpression d'une protéine ayant l'activité delta-5,7 stérol,delta-7 réductase et de P₄₅₀SCC et éventuellement de l'ADR et de l'ADX et on isole éventuellement la pregnénolone libre ou estérifiée.

L'invention concerne particulièrement le procédé ci-dessus dans lequel on cultive une levure transformée coexprimant une protéine ayant l'activité delta-5,7 stérol delta-7 réductase, le P₄₅₀SCC, l'ADR et l'ADX, tout particulièrement le procédé ci-dessus dans lequel la protéine ayant l'activité delta-5,7 stérol delta-7 réductase est la protéine d'A. thaliana delta-7Red et spécialement le procédé ci-dessus dans lequel la souche de levure est la souche EC01/pCD63.

Des exemples de production de pregnénolone selon l'invention sont donnés plus loin dans la partie expérimentale.

La levure transformée utilisée pour la réalisation du procédé de production de pregnénolone ci-dessus peut être par exemple une levure co-transformée par un vecteur d'expression de l'invention contenant une séquence d'ADN codant pour une protéine ayant une activité delta-5,7 stérol,delta-7 réductase et un vecteur d'expression du cytochrome P₄₅₀SCC et éventuellement de l'ADX et de l'ADR. Des vecteurs d'expression du cytochrome P₄₅₀SCC et/ou de l'ADX sont connus et des préparations sont décrites par exemple dans la demande de brevet européen EP 0340878 ainsi que plus loin dans la partie expérimentale.

La levure transformée utilisée peut être également une levure dans laquelle la séquence d'ADN codant pour la protéine ayant l'activité delta-5,7 stérol,delta-7 réductase est intégrée dans un locus particulier du génome, par exemple au locus ADE2 et dans laquelle l'ergostérol n'est plus le stérol majoritaire dans les conditions d'expression de la delta-7 réductase. La levure "intégrée" obtenue peut être ensuite transformée par des cassettes d'expression intégratives ou par des vecteurs d'expression contenant une séquence d'ADN codant pour le cytochrome P₄₅₀SCC et éventuellement pour l'ADX et l'ADR.

Un exemple de construction de souches de levure produisant in vivo la pregnénolone ou son ester acétique est donné plus loin dans la partie expérimentale.

L'invention a donc également pour objet une souche de levure transformée coexprimant une protéine ayant l'activité delta-5,7 stérol delta-7 réductase, le P₄₅₀SCC, l'ADR et l'ADX et accumulant de la pregnénolone libre ou estérifiée, particulièrement une souche de levure ci-dessus dans laquelle la protéine ayant l'activité delta-5,7 stérol delta-7 réductase est la protéine d'A. thaliana delta-7Red et spécialement une souche de levure dénommée EC01/pCD63 dont la construction détaillée est donnée plus loin dans la partie expérimentale.

L'invention s'étend aussi à une séquence d'ADN humain tel qu'obtenu selon le procédé de clonage défini ci-dessus, utilisé comme sonde pour diagnostiquer une déficience congénitale en delta-5,7 stérol,delta-7 réductase. La déficience en delta-5,7 stérol,delta-7 réductase comprend par exemple la déficience en 7-déhydrocholestérol réductase responsable de taux anormalement bas en cholestérol dans le plasma.

L'invention concerne également une méthode de détection de la déficience en delta-5,7 stérol,delta-7 réductase qui comprend l'incubation d'un échantillon contenant de l'ADN génomique humain avec la sonde définie ci-dessus dans des conditions d'hybridation standards et la mise en évidence de la fixation ou de l'absence de fixation de la sonde à l'ADN génomique, l'absence de fixation ou la réduction de celle-ci indiquant une déficience congénitale en delta-5,7 stérol, delta-7 réductase.

La méthode de l'invention peut permettre par exemple de détecter une déficience congénitale en delta-5,7 stérol, delta-7 réductase prénatale ou chez le nouveau-né ainsi que chez les patients atteints d'affections variées et notamment chez les patients ayant les manifestations cliniques du syndrome RSH/SLO.

Les figures ci-annexées illustrent certains aspects de l'invention :
La figure 1 représente le profil en stérols totaux extraits à partir du clone F22 résistant à la nystatine obtenu par criblage de la banque d'A. thaliana dans la levure FY 1679. L'analyse est faite par RP-HPLC à 205 nm ou à 285 nm (figure 1A) ou par CPG comparativement à la levure FY1679 non transformée (figure 1B).
La figure 2 représente une carte de restriction du fragment Not I du plasmide pF22 et la stratégie de séquençage.
La figure 3 représente la séquence nucléotidique de l'ADNc delta-7Red (SEQ ID N° 1) et la séquence en acides aminés correspondante (SEQ ID N° 2).
La figure 4 illustre la mesure in vitro de l'activité delta-5,7 stérol,delta-7 réductase d'une fraction microsomale de FY1679 transformée avec le vecteur d'expression inductible "delta-7/V8". L'analyse est faite par CPG et montre la transformation du substrat 7-déhydrocholestérol (TR=16,528) en cholestérol (TR=15,887) en présence des stérols endogènes ergosta 5,22 diène 3-ol (TR=16,682) ; ergostérol (TR = 17,249) ; ergosta 5-ène3-ol (TR=17,664).
La figure 5 illustre la production de pregnénolone in vitro par clivage d'ergosta 5-ène 3-ol (fig. 5A) ; d'ergosta 5,24(28) diène 3-ol (5B) ou d'ergosta 5,22 diène 3-ol (5C) purifié à partir d'une levure transformée exprimant la delta-7Red, puis incubé avec P₄₅₀SCC, ADX et ADR. L'analyse est faite par CPG comparativement à un clivage témoin de cholestérol (trait plein).
La figure 6 illustre la construction du plasmide intégratif pADΔ7 permettant l'intégration de l'ADNc codant pour la delta-7Red (stérol Δ7 réductase) au locus ADE2.
La figure 7 représente l'analyse par CPG des stérols totaux extraits par saponification alcaline de la souche FY1679 et de la souche intégrée ELR01 cultivées en présence de galactose.
La figure 8 schématise la construction du vecteur navette E. coli-S. cerevisiae V13 contenant un site unique SalI (Sa) dans le site de clonage multiple.
La figure 9 représente les étapes de la construction des plasmides intégratifs pCD62-1 et pCD62-2 qui permettent d'insérer la cassette d'expression ADR dans la région intergénique des gènes leu2 et spllΔ.
   SCM1 et SCM2 : sites de clonage multiples.
La figure 10 représente la stratégie de la construction de la souche CDR01.
La figure 11 représente les étapes de la construction du plasmide d'expression pCD63 contenant les deux cassettes d'expression ADX et P₄₅₀SCC.
La figure 12 représente l'analyse par CPG des stérols extraits à partir des cellules (lysat cellulaire) ou du milieu de culture (milieu) isolés à partir de souche EC01/pCD63 après une induction par le galactose de 9 h (figure 12a) ou de 24 h (figure 12b).
La figure 13 représente la structure du plasmide pTG 7457.
La figure 14 représente la structure du plasmide pTG 7453.
La figure 15 représente la structure du plasmide pTG 10014.
La figure 16 représente la structure du plasmide pTG 10004.
La figure 17 représente la structure du plasmide pTG 10031.
La figure 18 représente la structure du plasmide pTG 10033.

Les exemples suivants illustrent l'invention sans la limiter.

### EXEMPLE 1 : Clonage d'ADNc codant pour la delta-5,7 stérol, delta-7 réductase (delta-7Red) d'A. thaliana

### A - Criblage de la banque d'expression d'A. thaliana dans la levure

La banque d'expression d'ADNc de départ est la banque décrite par M. Minet et al. (Plant J., 2, 417-422, 1992) qui a été préparée à partir d'ARNm d'A. thaliana au stade de germination au stade à deux feuilles et dont les ADNc bordés par le site Not I ont été insérés au site Bst XI de la cassette d'expression du vecteur navette E. coli/S. cerevisiae pFL61. Cette cassette contient les séquences du promoteur et du terminateur du gène de la phosphoglycérate kinase (PGK). L'origine de réplication de la levure dérivée de la séquence 2µ et un marqueur de sélection URA3 assurent la propagation du vecteur dans la levure. La propagation du vecteur dans E. coli est dérivée du plasmide pUC19.

La souche de levure FY 1679 (Mata), qui est une souche isogénique de la souche S288C décrite par A. Thierry et al. (Yeast, 6, 521-534, 1990), a été transformée par la banque d'ADNc en utilisant la méthode à l'acétate de lithium décrite par D. Gietz et al. (Nucleic Acids Res., 20, 1425, 1992).

Les cellules ont été étalées sur un milieu synthétique SGI contenant 7 g/l de "yeast nitrogen base" (Difco), 1 g/l de bactocasaminoacides (Difco), 20 g/l de glucose, 20 mg/l de tryptophane et qui est dépourvu d'uracile. 10⁵ transformants prototrophes pour l'uracile ont été obtenus puis groupés et étalés à nouveau sur le même milieu synthétique dépourvu d'uracile et contenant 2 ou 5 µg/ml de nystatine à raison de 5x10⁴ cellules par boite. 10⁶ cellules pour chaque concentration en nystatine ont été ainsi criblées. Après 3 jours d'incubation à 28°C, environ 100 clones, qui ont poussé à la concentration de 2 µg/ml en nystatine, ont été récupérés en constituant des groupes de 5 clones dont la composition en stérols a été analysée par chromatographie liquide à haute performance en phase inversée (noté RP-HPLC dans ce qui suit) alors qu'un seul clone résistant, dénommé F22, a été obtenu à la concentration de 5 µg/ml en nystatine.

### B - Analyse des stérols accumulés dans le clone F22

Les stérols totaux de la levure sont préparés selon la méthode de saponification alcaline décrite par L. Parks et al. (Methods in Enzymol., 111, 333-346, 1985), puis analysés par RP-HPLC ou/et par chromatographie en phase gazeuse (noté CPG).

Le résidu de stérols obtenu est dissout dans le mélange éthanol-tétrahydrofurane-eau (65:10:25 v/v) puis analysé par RP-HPLC sur une colonne de silice greffée C18 Applied Biosystems (100 x 2,1 mm) au débit de 1 ml/mn et à 55°C à l'aide d'un gradient linéaire de méthanol dans de l'eau (50 % à 100 % sur 18 mn) et une détection photométrique à 205 nm et 285 nm comparativement à des standards d'ergostérol, campestérol et cholestérol.

La composition en stérols est aussi analysée par CPG sur une colonne capillaire SE-30 Alltech (30 m x 0,32 mm) avec de l'hélium comme gaz porteur, une température de 280°C et 310°C pour l'injecteur et le détecteur respectivement, avec une augmentation initiale de la température de 110°C à 245°C à la vitesse de 45°C/mn, puis de 3°C/mn pour atteindre 280°C.

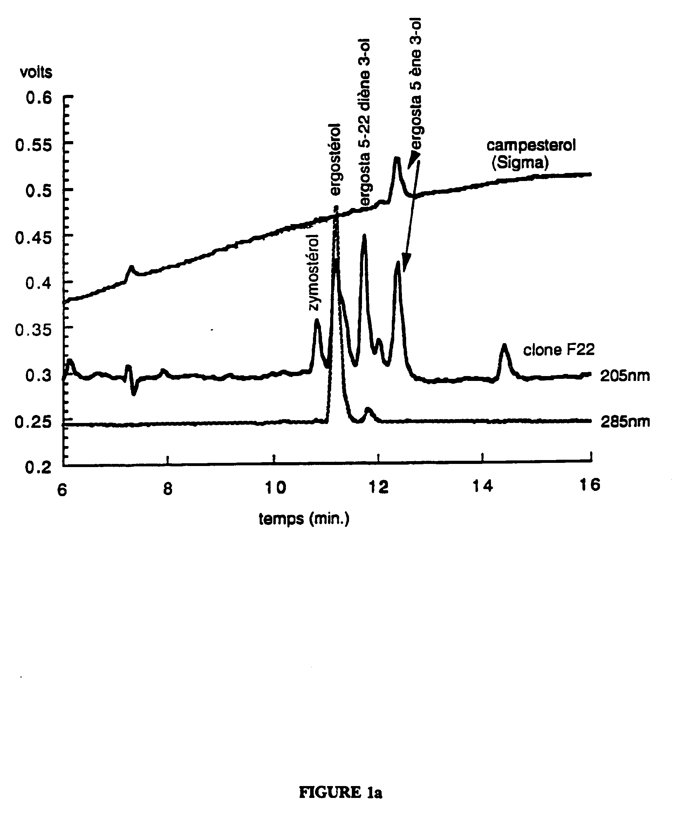

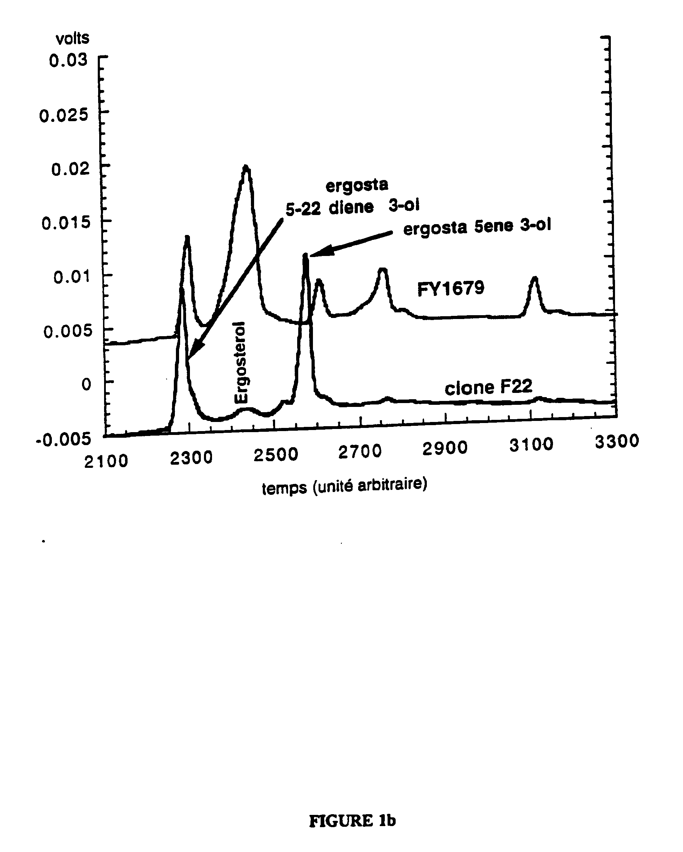

L'analyse par RP-HPLC (figure 1A) et l'analyse par CPG (figure 1B) montrent le profil des stérols accumulés dans le clone F22 obtenu ci-dessus caractérisé par la disparition presque complète de l'ergostérol, stérol majoritaire de la souche non transformée FY 1679, et son remplacement par deux stérols majeurs et en quantité analogue qui n'absorbent pas à 285 nm et ne possèdent donc plus une double insaturation conjuguée selon l'analyse par RP-HPLC.

A la figure 1A, le campestérol (Sigma) (24-R-ergosta 5-ène 3-ol) contient environ 35 % de dihydrobrassicastérol (24-S-ergosta 5-ène 3-ol).

### C - Clonage de l'ADNc delta-7Red

Les plasmides provenant du clone F22 ont été amplifiés dans E. coli selon la méthode décrite par J. N. Strathern et al. (Methods in Enzymol., 194, 319-329, 1991), puis digérés par Not I. Un fragment de 600 paires de bases (bp) environ et un fragment de 1,6 kbp ont été obtenus. La souche FY1679 a été transformée avec chacun des fragments ci-dessus respectivement. La composition en stérols de chaque clone de levure transformée a été analysée comme indiqué ci-dessus et a permis de distinguer le plasmide portant le gène responsable du profil modifié en stérols. Le plasmide ainsi identifié a été dénommé pF22.

### D - Détermination de la séquence de l'ADNc delta-7Red

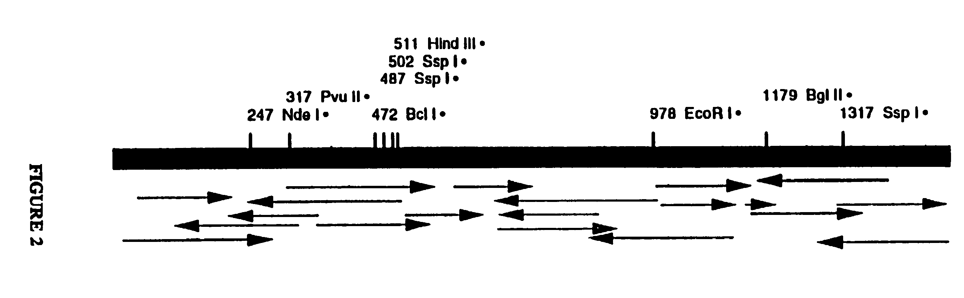

L'insert ADNc de pF22 a été sous-cloné au site Not I du vecteur pUC9N dérivé de pUC9 (Pharmacia) dans lequel le site Eco RI du site multiple de clonage est remplacé par insertion d'un site de restriction Not I tout en conservant la phase de lecture du gène LacZ. Une carte de restriction a été ensuite déterminée (figure 2).

Des fragments de restriction ayant le site externe Not I et les sites internes EcoRI, PvuII ou HindIII respectivement ont été sous-clonés dans le plasmide pBluescript (Stratagène). La séquence nucléotidique a été déterminée par la méthode de Sanger avec l'ADN polymérase Sequenase (kit Stratagène) sur les deux brins en utilisant les amorces directe et inverse de pUC9, T3 et T7 de pBluescript ou des amorces spécifiques déduites de la séquence nucléotidique de l'ADNc.

La compilation de l'ensemble des séquences obtenues donne la séquence nucléotidique de l'ADNc delta-7Red d'A. thaliana (SEQ ID N° 1) représentée à la figure 3. Elle comporte 1496 nucléotides se terminant par une séquence de polyadénylation. Elle présente une phase de lecture ouverte débutant par une méthionine initiatrice au nucléotide 76 et se terminant par un codon de terminaison au nucléotide 1366. Il en résulte une phase de lecture ouverte de 1290 nucléotides codant pour une protéine de 430 acides aminés. La région codante de l'ADNc delta-7Red code pour la protéine delta-7Red dont la séquence déduite en acides aminés (SEQ ID N° 2) est montrée à la figure 3. La séquence de la protéine comprend 430 acides aminés ayant une masse moléculaire calculée de 49,286 kDa.

Un échantillon de souche E. coli DH5-1 contenant l'ADNc delta-7Red dans le vecteur pUC9N (désigné ADNc delta7red/pUC9N) a été déposé à la CNCM le 10 février 1995 sous le numéro I-1535.

### E - Détermination de la séquence consensus SEQ ID N° 3

Par recherche informatisée dans les bases de séquences (Genbank et EMBL), il a été mis en évidence que la séquence de la protéine delta-7Red d'A. thaliana présente certaines similarités de séquence avec d'autres stérol réductases, en particulier la stérol C-14 réductase et la stérol C-24(28) réductase de S. cerevisiae décrites par R. T. Lorentz et al. (DNA Cell Biol., 11, 685-692, 1992) et W. Chen et al. (Yeast, 7, 305-308, 1991) respectivement ainsi que le produit du gène sts 1+ de S. pombe décrit par M. Shimanuki et al. (Mol. Biol. Cell, 3, 263-273, 1992) et de la stérol C-14 réductase de Neurospora crassa (N° X77955 dans la base EMBL). De plus, la protéine delta-7Red montre une similarité avec les 400 acides aminés de l'extrémité C-terminale du récepteur de la lamine B de poulet et avec celle du récepteur humain correspondant décrits par H. J. Worman et al. (J. Cell Biol., 111, 1535-1542, 1990) et E. Schuler et al. (J. Biol. Chem., 269, 11312-11317, 1994).

Des alignements d'identité de séquence ont été établis entre la séquence en acides aminés SEQ ID N° 2 déduite de l'ADNc delta-7Red obtenu ci-dessus et celles des trois stérol réductases de levure et des deux récepteurs de lamine B, puis une séquence consensus nouvelle ayant la séquence en acides aminés (SEQ ID N° 3) : dans laquelle Xaa en position 7 est Trp ou Tyr et Xaa en position 12 est His ou Lys,
a été définie pour préparer des oligonucléotides utilisables comme amorces pour amplifier par PCR de nouvelles séquences d'ADN génomique ou d'ADNc codant pour une protéine ayant une activité delta-5,7 stérol,delta-7 réductase.

### F - Expression de la protéine delta-7Red dans la levure.

### a) construction du vecteur d'expression delta-7/V8 inductible dans la levure

La délétion des régions non codantes de l'ADNc de pF22 a été effectuée par la technique d'amplification PCR en utilisant les oligonucléotides spécifiques suivants :
5'CGCGGATCCA TGGCGGAGAC TGTACATTC 3' (SEQ ID N° 4)
   et
5'CAGGGTACCT CAATAAATTC CCGGAATG 3' (SEQ ID N° 5)
qui ont été définis pour introduire un site de restriction BamH I immédiatement en amont du codon d'initiation et un site Kpn I immédiatement en aval du codon stop.

L'ADNc a été amplifié à partir de 1 ng de plasmide "ADNc delta7red/pUC9N" en présence de 2 unités de Pfu DNA polymérase et 0,2 µM de chacune des amorces ci-dessus en utilisant les conditions d'amplification suivantes : 94°C, 10s ; 52°C, 50s ; 74°C, 90s ; 33 cycles avec la trousse PCR de Stratagène.

Un fragment de 1300 bp environ a été obtenu, puis digéré par les enzymes de restriction BamH I et Kpn I et inséré aux sites BamH I et Kpn I du vecteur navette E. coli/S. cerevisiae pYeDP1/8-2 (dénommé V8) décrit par C. Cullin et al. (Gene, 65, 203-217, 1988). V8 porte le marqueur de sélection URA3 et contient une cassette d'expression dans la levure contenant le promoteur GAL10/CYC1 et la séquence terminateur du gène PGK. Le vecteur ainsi obtenu, dénommé delta-7/V8, permet l'expression inductible par le galactose de la protéine delta-7Red.

### b) Production de la protéine delta-7red

La souche de levure FY 1679 (Mata) a été transformée avec le plasmide delta-7/V8 obtenu ci-dessus en utilisant la méthode à l'acétate de lithium décrite par D. Gietz et al. (déjà cité).

La levure transformée a été cultivée à 27°C, en milieu sélectif SGI décrit ci-dessus mais dans lequel la concentration en glucose est de 5 g/l, jusqu'à l'obtention d'une saturation de densité cellulaire (DO₆₀₀ₙₘ=12). La culture est ensuite diluée par addition d'un volume de milieu complet YP (10 g/l en extrait de levure (Difco) et 10 g/l en bactopeptone (Difco)), puis addition d'éthanol (1,5 % v/v) comme source de carbone. Quand la croissance a permis d'atteindre une concentration cellulaire d'au moins 7x10⁷ cellules/ml, l'expression de la delta-7Red a été induite par addition de galactose à la concentration de 20 g/l.

L'induction a été aussi réalisée en milieu minimum sélectif SLI, qui correspond au milieu SGI dans lequel le glucose est remplacé par le galactose (20 g/l), jusqu'à l'obtention d'une concentration cellulaire de 2 x 10⁷ cellules/ml.

### c) Test enzymatique in vitro de l'activité delta-5,7 stérol, delta-7 réductase

L'expression de la protéine delta-7Red a été mise en évidence en utilisant un test enzymatique décrit par M. Taton et al. (Biochem. Bioph. Res. Commun., 181, 465-473, 1991) mais sans système de régénération du NADPH, avec des préparations cellulaires microsomales ou cytosoliques de la levure induite ci-dessus.

Les fractions cellulaires ont été obtenues par cassage mécanique de cellules induites et isolement des fractions par ultracentrifugation selon la méthode décrite par P. Urban et al. (Eur. J. Biochem., 222, 843-850, 1994). Les cellules sont collectées puis lavées deux fois avec le tampon TE-KCl (TrisHCl 50mM, pH 7,4 ; EDTA 1mM, KCl 0,1 M) et remises en suspension dans le tampon de lyse TE-sorbitol 0,6 M. Des billes de verre de 0,45-0,5 mm de diamètre (Braun) sont ajoutées jusqu'à affleurer la surface de la suspension cellulaire que l'on agite ensuite pendant 5 mn à 4°C. Le lysat cellulaire en surface est récupéré et les billes de verre sont lavées trois fois avec le tampon de lyse. Le lysat et les lavages sont réunis puis centrifugés à 20.000 g pendant 13 mn à 4°C de façon à éliminer les fractions mitochondriales. Le surnageant recueilli est centrifugé pendant 1 h à 100.000 g et à 4°C. Le culot qui contient la fraction microsomale et le surnageant qui représente la fraction cytosolique sont recueillis séparément.

La fraction microsomale ou la fraction cytosolique obtenues sont respectivement incubées pendant 90 mn à 37°C dans du tampon Tris/HCl 100 mM à pH 7,3 contenant comme substrat du 7-déhydrocholestérol 150 µM émulsifié avec du tween 80 (1,5 g/l) et en présence de NADPH 2 mM. Les stérols sont extraits par addition de 3 volumes de mélange méthanol-dichlorométhane (50:50, v/v) puis analysés en CPG comparativement aux produits standards.

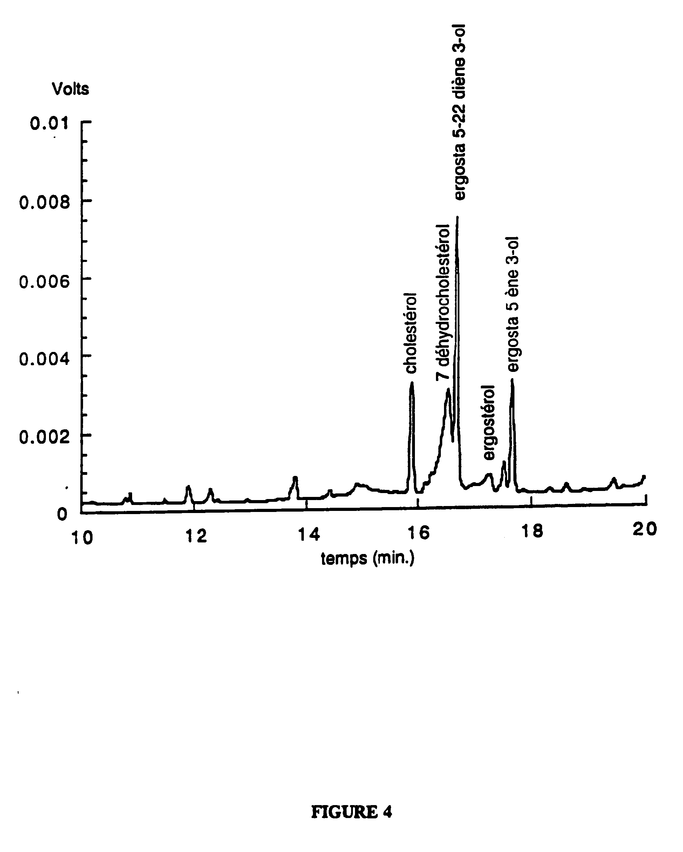

La formation de cholestérol (TR = 15,887 mn) à partir de 7-déhydrocholestérol (TR = 16,528 mn) est montrée à la figure 4 avec une fraction microsomale (3,5 mg/ml en protéine) obtenue ci-dessus à partir de la levure FY 1679 transformée avec le vecteur delta-7/V8, induite pendant 3 h et dont les stérols endogènes ont des temps de rétention supérieurs (TR = 16,682 mn pour l'ergosta 5-22 diéne 3-ol ;
TR = 17,249 mn pour l'ergostérol et TR = 17,664 mn pour l'ergosta 5-ène 3-ol).

Ces résultats montrent que la protéine delta-7Red, d'une part est exprimée dans la levure transformée, d'autre part possède une activité delta-5,7 stérol,delta-7 réductase.

### EXEMPLE 2 : Réduction in vivo de stérols endogènes de levure insaturés en position C-7

Des souches de levure dont les stérols majoritaires ont une double liaison en position C-5,7 ont été transformées avec le vecteur delta-7/V8 obtenu à l'exemple 1, puis cultivées et induites comme indiqué à l'exemple 1. Les stérols endogènes, dont le profil est analysé par CPG, ont été extraits et purifiés par RP-HPLC comme indiqué à l'exemple 1 en utilisant une colonne C18 préparative (100 X 4,6 mm), puis identifiés par IR, UV, SM et RMN. Les trois souches suivantes ont été respectivement utilisées :
- La souche FY1679 décrite à l'exemple 1 ;
- La souche mutant erg5, dénommée PLC 1051, caractérisée par une déficience en stérol C-22 désaturase, qui a été construite par croisement entre la souche FY1679 et la souche originale pol5 décrite par S. W. Molzahn et al. (J. Gen. Microbiol., 72, 339-348, 1972) et qui accumule de l'ergosta 5,7-diène 3-ol.
- La souche double mutant erg4,5, dénommée PLC 1451, caractérisée par une déficience en stérol C-22 désaturase (erg5) et stérol C-24(28) réductase (erg4), a été obtenue par croisement entre la souche FY1679 et une souche pol5 décrite par S. W. Molzahn et al. (déjà cité) ayant acquis une résistance spontanée à la nystatine au cours d'un criblage systématique de levures à la recherche de mutants de stérols et qui accumule de l'ergosta 5,7,24(28) triène 3-ol. La souche haploïde résultante, qui porte la double mutation erg4, erg5, croît en présence de galactose et de substrat non fermentable et est auxotrophe pour l'uracile, le tryptophane et l'histidine. Les souches PLC 1051 et PLC 1451 ont été déposées à la CNCM le 10 février 1995 sous les numéros I-1536 et I-1537 respectivement.
Les stérols réduits majeurs identifiés respectivement à partir des trois souches transformées ci-dessus sont indiqués dans le tableau suivant :

| Souche initiale | Stérol endogène majeur initial | Stérol endogène majeur réduit en C-7 |
|---|---|---|
| FY 1679 | ergosta 5,7,22 triène 3-ol (ergostérol) | ergosta 5-ène 3-ol (dihydrobrassicastérol) ergosta 5,22 diène 3-ol (brassicastérol) |
| | | |
| erg5 | ergosta 5,7 diène 3-ol | ergosta 5-ène-3-ol |
| PLC 1051 | | |
| | | |
| erg4,5 | ergosta 5,7,24(28)triène- | ergosta 5,24(28) diène- |
| PLC 1451 | 3-ol | 3-ol (ostréastérol) |

### EXEMPLE 3 : Production de pregnénolone in vitro par clivage de stérols endogènes de levure réduits en C-7

La production de pregnénolone est réalisée en utilisant le test enzymatique de clivage de la chaîne latérale du cholestérol in vitro décrit par Wada et al. (Arch. Biochem. Biophys., 290, 376-380, 1991) dans lequel 260 µM d'un stérol réduit en C-7 obtenu à l'exemple 2 sont incubés dans 150 µl de tampon phosphate 10 mM, pH 7,4, NaCl 100 mM, tween 20 0,3 % en présence de 140 nM d'adrénodoxine réductase, de 1,16 µM d'adrénodoxine et de 0,68 µM de cytochrome P₄₅₀SCC d'origine bovine obtenus à partir de glandes surrénales par exemple selon D. W. Seybert et al., J. Biol. Chem., 254, 12088-12098, 1979.

La réaction est déclenchée par addition de 150 µM de NADPH. Après une incubation de 80 mn à 37°C, la réaction est arrêtée par addition d'un volume de mélange méthanol-dichlorométhane (50:50 v/v). Les stérols sont extraits et analysés par CPG comme indiqué à l'exemple 1.

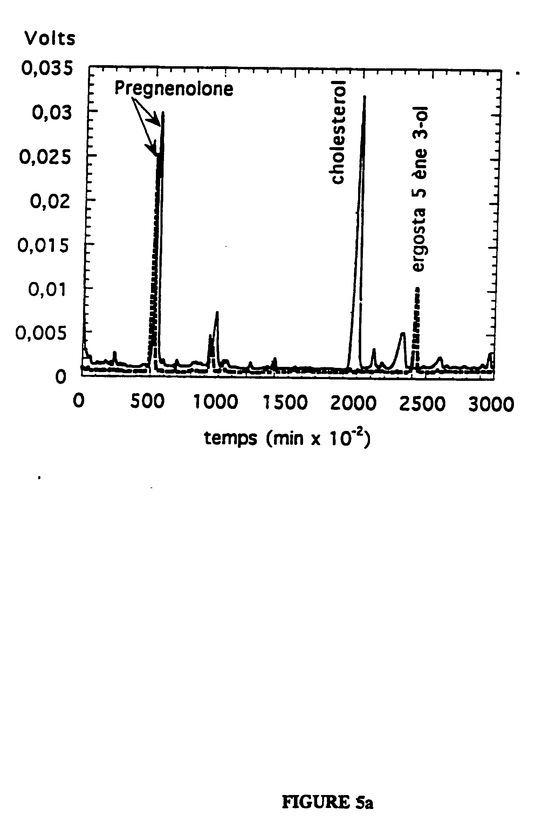

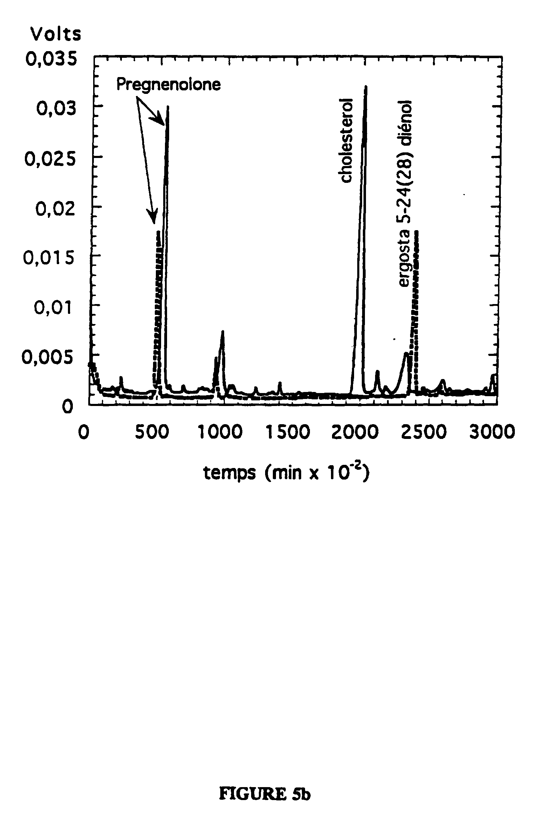

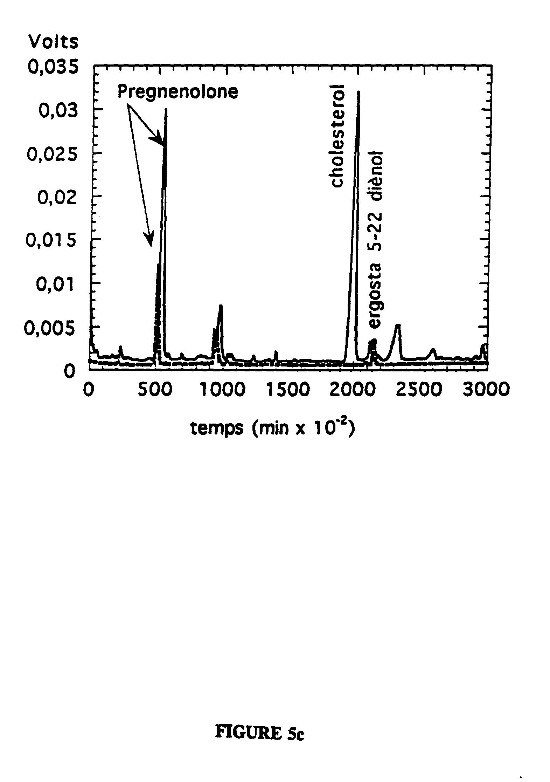

La figure 5 montre respectivement que l'ergosta 5-ène 3-ol (fig. 5A), l'ergosta 5,24(28) diène 3-ol (fig. 5B) ou l'ergosta 5,22 diène 3-ol (fig. 5C) est substrat du cytochrome P₄₅₀SCC et conduit à un produit ayant un TR identique à celui de la pregnénolone obtenue dans les mêmes conditions par clivage du cholestérol.

Les résultats obtenus montrent qu'une levure transformée exprimant la delta-7Red accumule des stérols utilisables directement pour préparer de la pregnénolone par oxydation biologique in vitro.

### EXEMPLE 4 : Construction de souches de levure produisant in vivo la pregnénolone ou son ester acétique

### A - Construction de la souche ELR01 contenant une cassette d'expression pour la delta-7Red d'A. thaliana intégrée dans le locus ADE2 de la souche haploïde FY1679 mating type a (FY1679 Mata)

### a) Construction du plasmide intégratif pADdelta-7 (pADA7) :

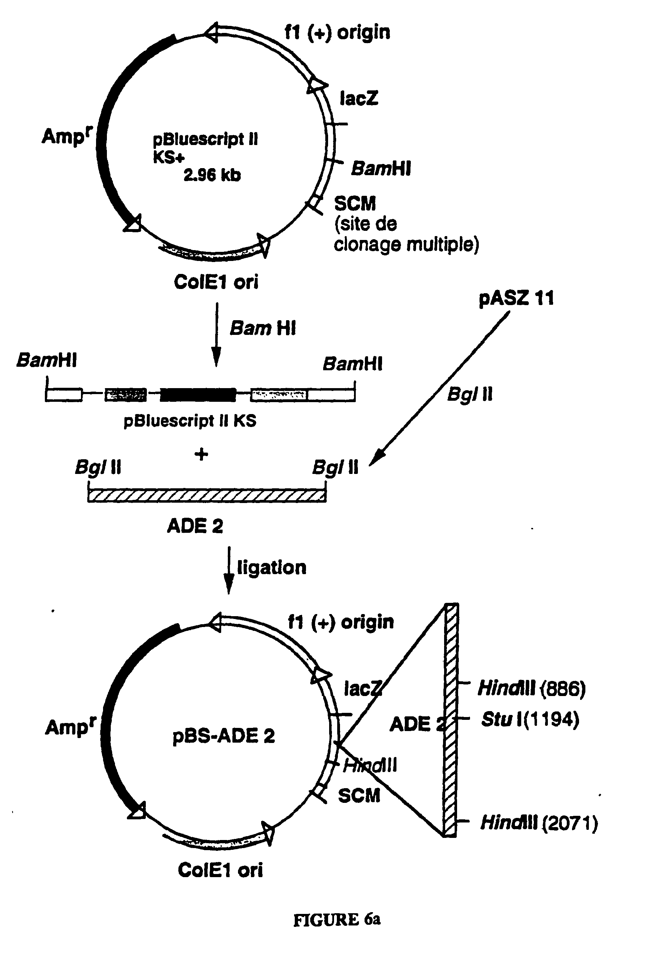

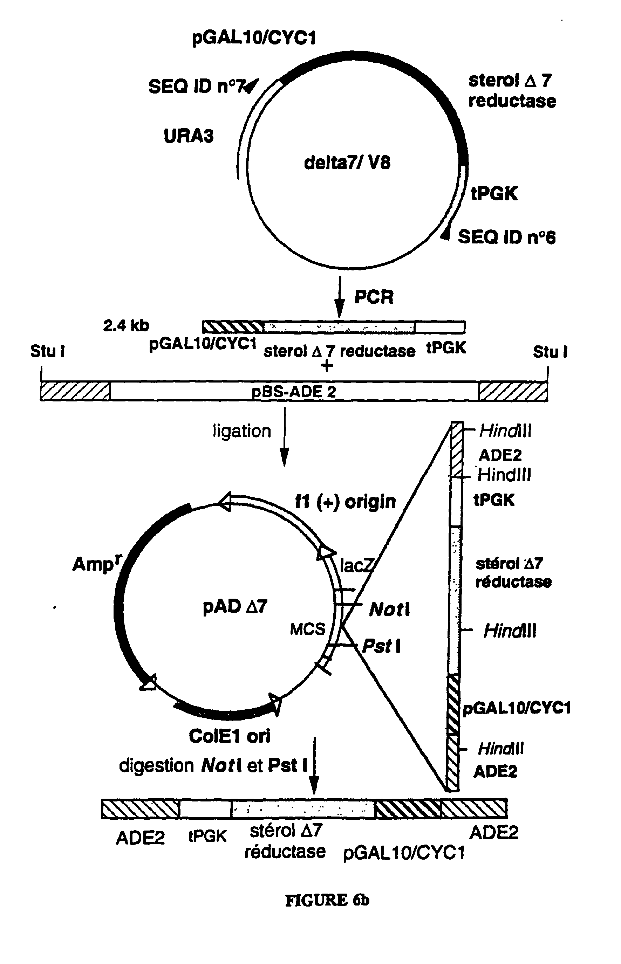

La construction du plasmide pADA7 a été menée comme décrit à la figure 6. Le fragment BglII (2244pb) contenant le gène ADE2 de S. cerevisiae a été isolé du plasmide pASZ 11 (A. Stotz et al., Gène, 95, 91, 1990) et inséré dans le vecteur pBluescriptII KS+ (Stratagène) au site BamHI du site de clonage multiple.

Le plasmide obtenu, dénommé pBS-ADE2, a été ensuite linéarisé à son site Stu I unique et déphosphorylé.

Un fragment de 2,44 kb environ contenant le promoteur GAL10/CYC1, la phase codante de la delta-7Red (stérol Δ7 réductase) et le terminateur PGK (tPGK) a été obtenu à partir du plasmide delta-7/V8, obtenu à l'exemple 1F par la technique de PCR en utilisant comme amorces les oligonucléotides suivants
5' GATTACGCCA AGCTTTTCGA AAC 3' (SEQ ID N° 6)
et
5' AGATCTTGAG AAGATGCGGC CAGCAAAAC 3' (SEQ ID N° 7)
qui ont été définis pour s'apparier respectivement avec l'extrémité 3' de tPGK et l'extrémité 3' du gène URA3. On a utilisé le plasmide delta-7/V8 comme matrice (80 ng), les oligonucléotides ci-dessus (0,5 µM chacun), la Pfu DNA polymérase native (1 unité dans le tampon recommandé par Stratagène) et les conditions d'amplification suivantes : 35 cycles ; 1 min à 95°C ; 5s à 95°C ; 30s à 56°C ; 4 min 30s à 70°C). Le fragment d'amplification obtenu a ensuite été cloné à bouts francs dans le plasmide pBS-ADE2 linéarisé ci-dessus pour donner le plasmide pADA7 dans lequel le fragment NotI-PstI de 4720 pb environ porte le gène ADE2 interrompu par la cassette d'expression de la delta-7Red.

### b) Intégration chromosomique dans la souche de levure FY1679 Mata :

Le fragment NotI-PstI( 4720 bp), isolé à partir du plasmide pADΔ7 digéré avec les enzymes de restriction NotI et PstI, a été introduit dans la levure FY1679 Mata par transformation en utilisant la méthode à l'acétate de lithium décrite par D. Gietz et al. (déjà cité).

Les transformants ayant intégré ce fragment par recombinaison homologue ont été sélectionnés par leur résistance à la nystatine, ce phénotype étant dû à l'expression de la delta-7Red qui convertit les delta-5,7 stérols de la levure en delta-5 stérols.

Les cellules transformées ont été incubées à 28°C pendant 4 heures en milieu complet YP décrit à l'exemple 1F contenant du glucose (20 g/l) et supplémenté en adénine (20 mg/l). Elles ont été ensuite concentrées puis étalées sur un milieu minimum SLI-agar (1g/l de bactocasaminoacides ; 7 g/l de "yeast nitrogen base" ; 20 g/l de galactose ; 20 mg/l d'adénine ; 20 g/l d'agar) et incubées pendant une nuit à 28°C pour induire l'expression du gène de la delta-7Red. L'absence de complémentation en uracile permet de limiter la croissance des cellules. Les clones ont été récupérés, groupés puis étalés sur des boites sur un milieu complet YP contenant du galactose (20 g/l), supplémenté en adénine (20 mg/l) et en présence de concentrations croissantes de nystatine (0 µg/ml, 1 µg/ml, 2 µg/ml, 5 µg/ml, 20 µg/ml respectivement). Au 4ème jour, une vingtaine de clones ayant poussé à la concentration de 5 µg/ml en nystatine ont été obtenus. Douze de ces clones ont été repiqués sur des boites en milieu minimum WO (7g/l de "yeast nitrogen base" sans acides aminés, 20 g/l de glucose) enrichi en uracile, leucine, tryptophane, histidine et adénine (20 mg/l chacun).

L'auxotrophie pour l'adénine due à la disruption du gène ADE2 a été ensuite confirmée en observant l'absence de croissance sur le milieu minimum WO décrit ci-dessus enrichi en uracile, leucine, tryptophane, histidine mais dépourvu d'adénine.

La présence de la cassette d'expression dans le génome de la levure a été contrôlée par amplification par PCR à partir de l'ADN génomique des clones obtenus et en utilisant les amorces ayant les séquences SEQ ID N° 6 et SEQ ID N° 7 ci-dessus.

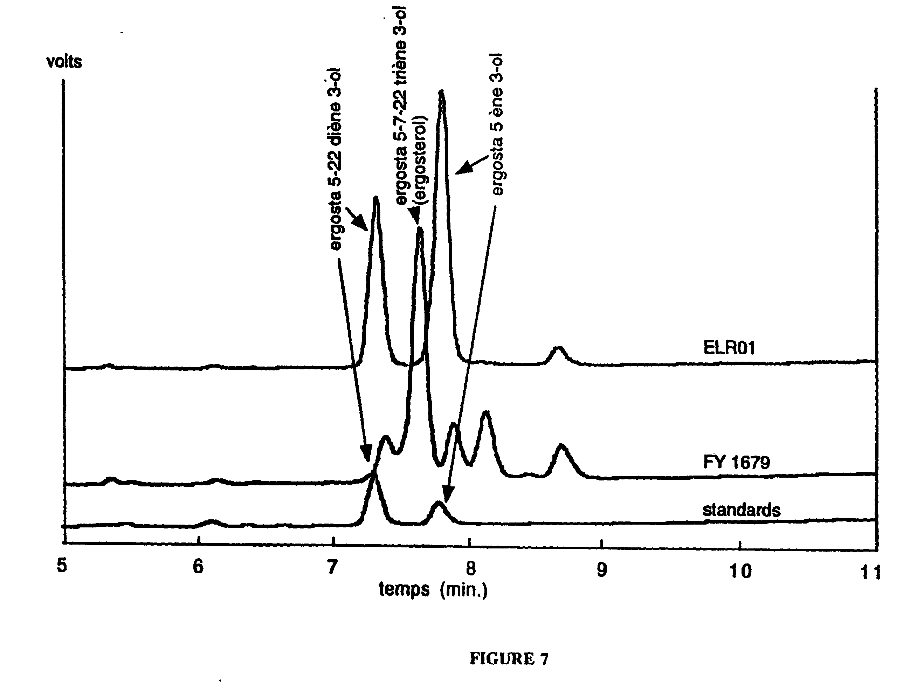

La fonctionnalité du gène delta-7Red intégré a été confirmée par l'analyse par CPG de la composition en stérols accumulés dans la levure et extraits par saponification alcaline selon le mode opératoire décrit à l'exemple 1B avec une colonne capillaire SE 30 de cinq mètres (Alltech). L'analyse montre un profil modifié comprenant des stérols saturés en C-7 lorsque les clones sont cultivés en présence de galactose. La souche obtenue,dénommée ELR01, accumule de l'ergosta 5 ène 3-ol et de l'ergosta 5,22 diène 3-ol au lieu d'ergosta 5, 7, 22 triène 3-ol (ergostérol),stérol majoritaire de la souche FY1679 initiale comme cela est montré à la figure 7.

La souche ELR01 ainsi construite exprime le gène de la delta-7Red lorsqu'elle est cultivée en présence de galactose du fait du contrôle transcriptionnel par le promoteur GAL10/CYC1. Bien que l'unité d'expression pour la delta-7Red ait le même sens de transcription que le gène ADE2, aucune expression de la delta-7Red n'est détectable par analyse de la composition en stérols par CPG lorsque la culture est effectuée en présence de glucose, par suite de la répression du promoteur GAL10/CYC1.

### B - Construction de la souche CDR01 contenant une cassette d'expression pour la forme mature de l'adrénodoxine réductase bovine (ADRm) intégrée entre les loci LEU2 et SPL1 de la souche haploïde FY1679 mating type alpha (mat alpha).

### a) Construction du vecteur navette E. coli-s. cerevisiae V13

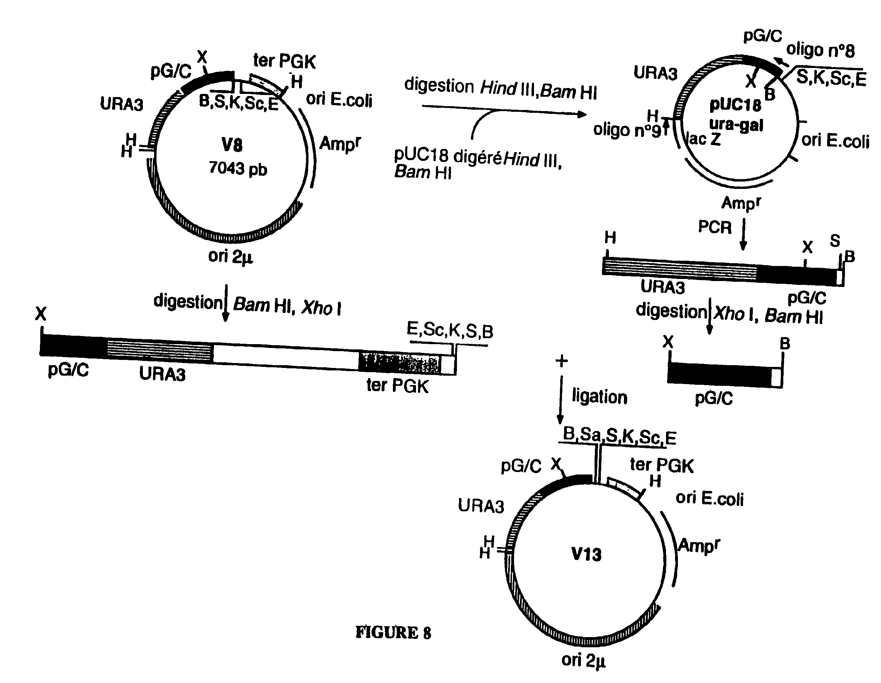

Le vecteur V13 correspond au vecteur V8 (C. Cullin et al., déjà cité) qui porte le marqueur de sélection URA3 et une cassette d'expression dans la levure contenant le promoteur GAL10/CYC1 (pG/C) et dans lequel un site SalI (Sa) supplémentaire a été introduit dans le site de clonage multiple, selon le schéma de construction donné à la figure 8.

Le vecteur V8 a été digéré par les enzymes de restriction HindIII et BamHI et le fragment BamHI-HindIII obtenu (1722 pb) contenant le gène URA3 et le promoteur GAL10/CYC1 (noté plus loin "ura-gal"), a été sous-cloné entre les sites correspondants du vecteur pUC18 (Pharmacia) digéré par les enzymes de restriction HindIII et BamHI.

Le fragment "ura-gal" a été ensuite amplifié par PCR à partir de 30 ng du plasmide obtenu pUC18/"ura-gal" dénaturé pendant 30s à 95°C en utilisant les conditions suivantes : 30 cycles ; 5s à 86°C ; 10s à 95°C ; 40s à 38°C ; 5s à 55°C et 2 min à 74°C), 2 unités de Taq DNA polymérase (Boehringer) dans le tampon du fabricant et 1µM de chacune des amorces ayant les séquences nucléotidiques suivantes :
5' GGGGATCCGT GGTCGACGTA ATTTAGTGTG TGTATTTGTG TTTGCG 3' (SEQ ID N° 8)
   et
5' GTAAAACGAC GGCCAGT 3' (SEQ ID N° 9)
L'amorce SEQ ID N° 8 contient un site BamHI GGATCC identique a celui du fragment "ura-gal", 3 nucléotides consécutifs au site BamHI ne s'hybridant pas à la matrice, un site SalI GTCGAC et une séquence homologue à celle du promoteur GAL10/CYC1. L'amorce SEQ ID N° 9 s'apparie à la séquence précédant le site Hind III du site de clonage multiple de pUC18. Le fragment HindIII-BamHI de 1722 pb obtenu après amplification a été digéré par XhoI et Bam HI libérant un fragment de 254 pb contenant le promoteur GAL10/CYC1 (pG/C) qui a été ensuite sous-cloné dans le vecteur V8 digéré par les enzymes de restriction BamHI et XhoI.

Le vecteur V13 résultant contient les sites de restriction permettant de sous-cloner aisément les ADNc codant pour l'adrénodoxine réductase bovine mature (ADRm), l'adrénodoxine bovine mature(ADXm) et le cytochrome P₄₅₀SCC bovin.

A partir du vecteur V13,le vecteur V13-ADR,le vecteur V13-ADX et le vecteur V13-SCC10 ont été respectivement préparés de la façon suivante :
**α) Construction du vecteur V13-ADR**
   Un fragment SalI-KpnI de 1478 pb portant l'ADNc codant pour ADRm a été isolé du plasmide pGBADR-2 décrit à l'exemple 25 de la demande de brevet européen EP 340878 et sous-clone dans les sites correspondants du vecteur V13 pour donner le vecteur V13-ADR.
**β) Construction du vecteur V13-ADX**
   Un fragment SalI BamHI de 390 pb portant l'ADNc codant pour ADXm a été isolé du plasmide pGBADX-1 décrit à l'exemple 23 de la demande de brevet européen EP 340878 et sous cloné dans les sites correspondants du vecteur V13 pour donner le vecteur V13-ADX.
**γ) Construction du vecteur V13-SCC10**
   Un fragment SalI-EcoRI de 1554 pb portant l'ADNc codant pour P₄₅₀SCC a été isolé du plamide pGBSCC-10 décrit à l'exemple 6 de la demande de brevet européen EP 340878 et sous-cloné dans les sites correspondants du vecteur V13 pour donner le vecteur V13-SCC10.

### b) Construction des plasmides intégratifs pCD62-1 et pCD62-2 :

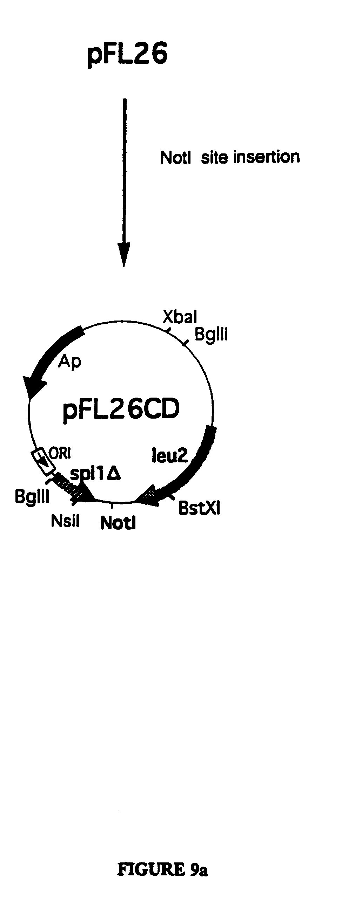

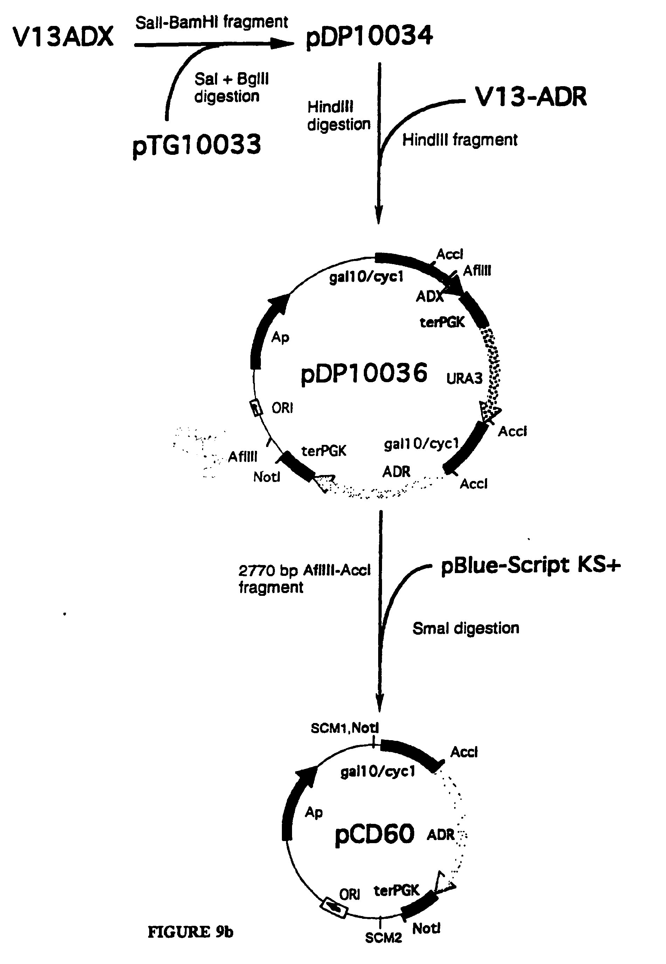

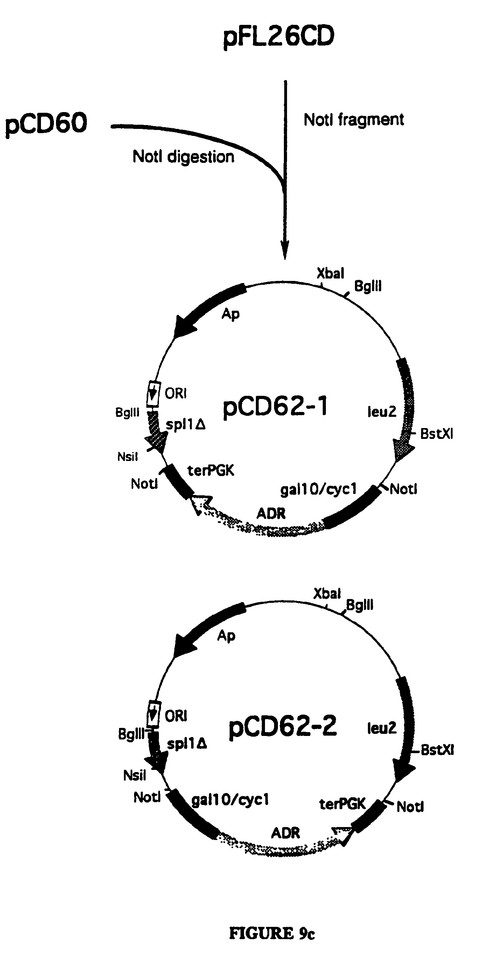

La construction des plasmides pCD62-1 et pCD62-2 a été menée comme décrit à la figure 9.

### α) Construction du plasmide pFL26CD

Un site NotI a été introduit dans le plasmide pFL26 (N. Bonneaud et al., Yeast, 7, 609-615, 1991), dans la région intergénique séparant le gène leu2 de l'extrémité 5' du gène spl1 (notée spllΔ) (C. Kolman et al., J. Bacteriol., 175, 1433, 1993) selon le mode opératoire suivant :

Deux fragments d'ADN de 704 pb et 347 pb portant respectivement l'extrémité 5' de leu2 et l'extrémité 3' de SpllΔ ont été synthétisés par PCR à l'aide des amorces ayant les séquences nucléotidiques suivantes :
5' TTGAAGGTTC AACATCAATT GATTG 3' (SEQ ID N° 10)
   et
5' GTGTGGCGGC CGCCTCCTTG TCAATATTAA TGTTAAAG 3' (SEQ ID N° 11)
   pour l'amplification du fragment de 704 pb et les séquences nucléotidiques
5' CAAGGAGGCG GCCGCCACAC AAAAAGTTAG GTGT 3' (SEQ ID N° 12)
   et
5' TCTGCTTCCC TAGAACCTTC TTATG 3' (SEQ ID N° 13)
   pour l'amplification du fragment de 347 pb.

Les amorces SEQ ID N° 11 et SEQ ID N° 12 possèdent une séquence GGCGGCCG qui correspond à un site NotI et dans laquelle 3 bases ne s'apparient pas avec la matrice. L'amorce SEQ ID N° 10 s'apparie à une séquence située 536 pb en amont du codon stop de leu2 et l'amorce SEQ ID N° 13 à une séquence située 194 pb en amont de celui de spllΔ.

Les fragments de 704 pb et de 347 pb sont d'abord amplifiés par PCR en utilisant comme matrice le plasmide pFL26 et comme enzyme la Pfu DNA polymérase dans les conditions standards décrites par le fournisseur (Stratagène).

Les deux fragments amplifiés obtenus s'apparient sur 20 pb au niveau des extrémités générées par l'amorce SEQ ID N° 11 (fragment 704 pb) et l'amorce SEQ ID N° 12 (fragment 347 pb) à partir de l'extrémité 5' ; ces 20 pb correspondent respectivement aux 20 premiers nucléotides de chacune de ces amorces.

Le produit résultant de l'appariement des fragments d'ADN de 704 pb (1 ng) et de 347 pb (2 ng) a été amplifié en utilisant les amorces SEQ ID N° 10 et SEQ ID N° 13 et les conditions suivantes : 30 cycles de 10s à 95°C, 5s à 60°C, 1. min à 45°C, 5s à 65°C et 2 min à 72°C suivis d'un cycle de 7 min à 72°C ; 50 pmol de chaque amorce et 1 unité de Pfu DNA polymerase dans 50 µl de tampon de réaction (Stratagène). Un fragment amplifié de 1031 pb contenant un site de coupure NotI a été obtenu. Ce fragment a été ensuite digéré par les enzymes BstXI et NsiI et le fragment de 920 pb obtenu, contenant le site NotI, a été inséré à la place du fragment BstXI-NsiI initial de pFL26 pour donner le plasmide pFL26CD, dont la carte est représentée à la figure 9a.

### β) Construction du plasmide pCD60 - Préparation du plasmide pDP10036 :

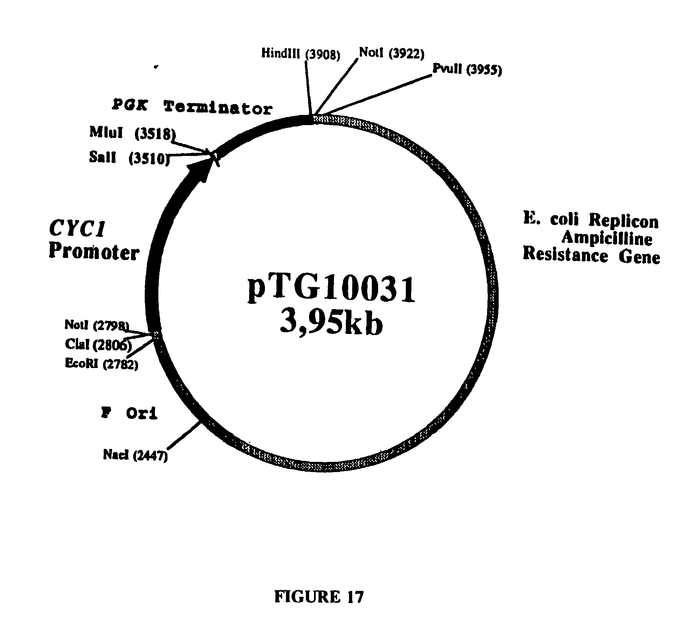

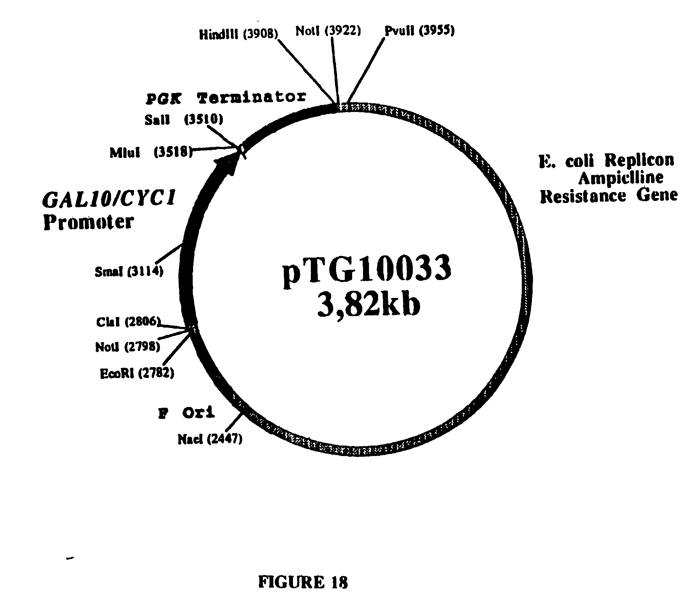

Un fragment SalI-BamHI de 390 pb portant l'ADNc codant pour l'adrénodoxine bovine mature (ADXm) a été isolé du plasmide V13-ADX puis sous-cloné dans les sites SalI-BglII du site de clonage multiple du plasmide pTG10033 qui est flanqué du promoteur inductible GAL10/CYC1 et du terminateur ter PGK. Le plasmide pTG10033, dont la carte est représentée à la figure 18 et qui correspond au vecteur d'expression pTG10031 (figure 17), contenant le promoteur CYC1 et terPGK, dans lequel le promoteur CYC1 a été remplacé par le promoteur GAL10/CYC1 a été préparé selon le mode opératoire décrit plus loin.

Le plasmide ainsi obtenu, dénommé pDP10034, porte la cassette d'expression ADX, c'est-à-dire le gène codant pour ADxm sous le contrôle transcriptionnel de GAL10/CYC1 et terPGK. Par la suite, le terme "cassette d'expression" sera employé pour tout gène sous la dépendance transcriptionnelle de GAL10/CYC1 et terPGK.

Un fragment HindIII de 3593 pb portant le marqueur de sélection URA3 et la cassette d'expression ADR a été isolé à partir du plasmide V13-ADR digéré par l'enzyme de restriction HindIII puis inséré dans le site correspondant du plasmide pDP10034 digéré par l'enzyme de restriction HindIII. Le plasmide obtenu, dénommé pDP10036, contient les cassettes d'expression ADX et ADR séparées l'une de l'autre par le marqueur URA3 (figure 9b).

### - Préparation du plasmide pCD60 :

Le fragment AflIII-AccI de 2770 pb portant la cassette ADR a été isolé par digestion partielle du plasmide pDP10036 avec les enzymes de restriction AflIII et AccI, rendu bouts francs par traitement avec le fragment de klenow de l'ADN polymérase **I** et sous-cloné après ligation à bouts-francs dans le site SmaI du plasmide pBlue-Script KS+ (Stratagène). Dans le plasmide obtenu, dénommé pCD60, la cassette d'expression ADR est encadrée de part et d'autre d'un site NotI, l'un se situant à 209 pb en amont du site de ligation AflIII/SmaI et provenant du fragment sous-cloné et l'autre provenant du site de clonage multiple (SCM1) de pBlue-Script KS+ (figure 9b).

### γ) Construction des plasmides pCD62-1 et pCD62-2 :

Le fragment NotI de 2558 pb, isolé à partir du plasmide pCD60 digéré par l'enzyme de restriction NotI, a ensuite été sous-cloné dans le site unique NotI du plasmide pFL26CD. Selon le sens de l'insertion du fragment, deux plasmides ont été obtenus, dénommés pCD62-1 et pCD62-2 (figure 9c).

Dans le plasmide pCD62-1, la cassette d'expression ADR est orientée dans le sens de la transcription du gène leu2 alors que cette orientation est inversée dans le plasmide pCD62-2.

Le plasmide pCD62-1 a été retenu pour les constructions ultérieures.

### c) Intégration chromosomique dans la souche de levure FY1679 (Matalpha) :

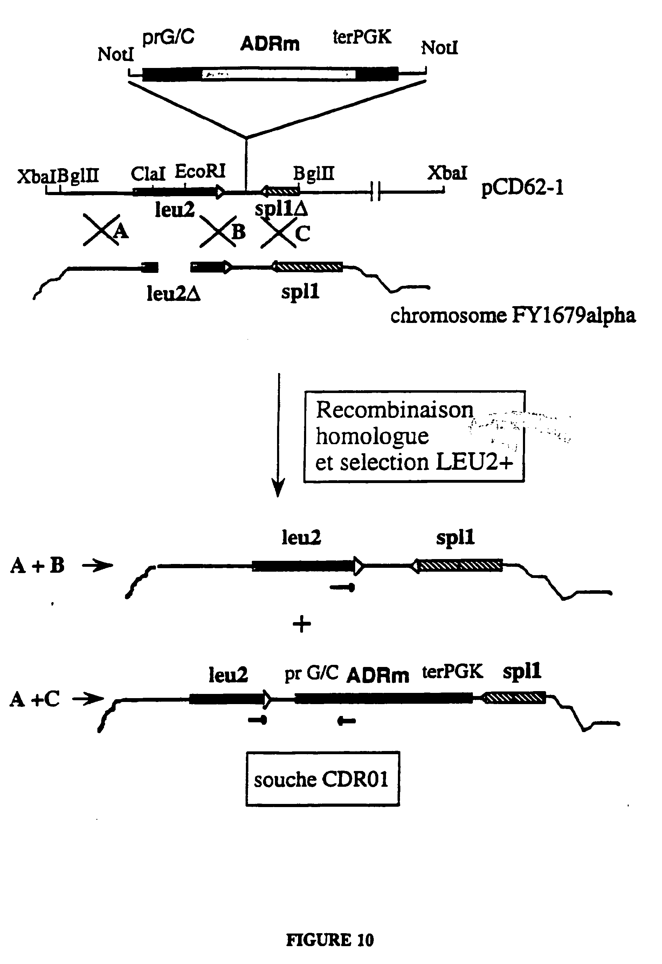

Le plasmide pCD62-1 contient des régions homologues à un locus chromosomique de la souche FY1679. Ces régions correspondent respectivement aux fragments BglII-ClaI de 1060 pb (A), EcoRI-NotI de 707 pb(B) et NotI-BglII de 602 pb (C) indiqués sur la figure 10.

La région correspondant au fragment ClaI-EcoRI de 486 pb du plasmide pCD62-1 a été délétée dans la souche FY1679, impliquant une auxotrophie de cette souche vis-à-vis de la leucine (souche LEU2⁻) (R. S. Sikorski et al., Genetics, 122, 19, 1989).

Le plasmide pCD62-1 a été linéarisé par digestion avec l'enzyme de restriction XbaI dont le site de coupure est situé à l'extérieur des régions homologues,puis a été introduit par transformation dans la souche FY1679 (Matalpha) en utilisant la méthode à l'acétate de lithium (D. Gietz et al., déjà cité).

La capacité de réparation cellulaire de l'ADN par la levure ("gap repair") et la sélection de recombinants ayant le phénotype LEU2⁺ ont permis de sélectionner dans un premier temps deux types de recombinants : le 1er type est obtenu après recombinaisons homologues au niveau des fragments A et B, le 2ème type est obtenu après recombinaisons homologues au niveau des fragments A et C. Seul ce dernier type de recombinaison a permis l'intégration de la cassette d'expression ADR en plus de la restauration du phénotype LEU2⁺.

Pour sélectionner ce 2ème type de clone, un criblage par PCR a été réalisé en utilisant l'amorce SEQ ID N° 10 ci-dessus et l'amorce ayant la séquence nucléotidique suivante :
5' TACATTAGGT CCTTTGTAGC 3' (SEQ ID N° 14)
de façon à confirmer à la fois la présence et la localisation correcte de la cassette d'expression ADR dans le génome de la souche FY1679 (Matalpha).

Dans ce criblage, l'amorce SEQ ID N° 14 s'apparie exclusivement à la séquence codante pour ADRm et l'amorce SEQ ID N° 10 s'apparie à une séquence chromosomique (figure 10).

La réaction d'amplification a été réalisée en utilisant comme matrice l'ADN génomique (20 ng) isolé de la souche FY1679 (Matalpha) (C. Hoffman et al., Gene, 57, 267, 1987), la Taq DNA polymérase (1U, Boehringer), 50 pmol de chaque amorce et 30 cycles de PCR (10s à 95°C, 1 min à 55°C, 3 min à 72°C) dans les conditions standards décrites par le fournisseur.

L'amplification a conduit à l'isolement d'un fragment de 2,9 kb correspondant au cas où la cassette d'expression a été intégrée. Dans le cas contraire, aucun produit d'amplification n'a été détecté.

La souche FY1679 (Matalpha) ainsi sélectionnée contenant la cassette d'expression ADR intégrée a été dénommée CDR01.

L'expression d'ADR par cette souche a été mise en évidence à partir de fractions cellulaires cytosoliques préparées selon le protocole décrit à l'exemple 1F, par immunodétection de la protéine reconnue par des anticorps anti-ADR.

La fonctionnalité de l'ADR exprimée par la souche CDR01 a été confirmée dans le test de clivage enzymatique de la chaîne latérale du cholestérol in vitro décrit à l'exemple 3 et dans lequel l'ADR purifiée (0,28 pmol) a été remplacée par une fraction cellulaire cytosolique de la souche CDR01 contenant 100 µg de protéines totales. Un taux de bioconversion du cholestérol en pregnénolone d'environ 25 % a été observé, comparable à celui obtenu avec l'ADR purifié.

### C - Construction de la souche diploïde EC01 coexprimant la delta-7Red d'A. Thaliana et l'ADRm.

La souche diploïde EC01 a été obtenue par croisement des souches haploïdes CDR01 et ELR01 obtenues ci-dessus selon le protocole décrit par G. Sprague et al. (Methods in Enzymology, 194, 77, 1991) :

Une première sélection sur milieu minimum WO décrit précédemment, enrichi en uracile, tryptophane et histidine (20 mg/l chacun) mais dépourvu en leucine, a permis d'isoler des clones diploïdes LEU2⁺ (caractère de prototrophie qui témoigne de la présence de la cassette d'expression ADR). Ces clones ont ensuite été testés pour leur résistance à la nystatine à 5 µg/ml (caractère de résistance qui témoigne de la présence de la cassette d'expression de la delta-7Red) sur le milieu synthétique solide SLI-agar décrit précédemment.

Une souche, dénommée EC01, a été ainsi isolée arbitrairement parmi les clones résistants à la nystatine.

### D - Construction de la souche EC01/pCD63 productrice de pregnénolone et de pregnénolone 3-acétate.

### a) construction du plasmide d'expression pCD63

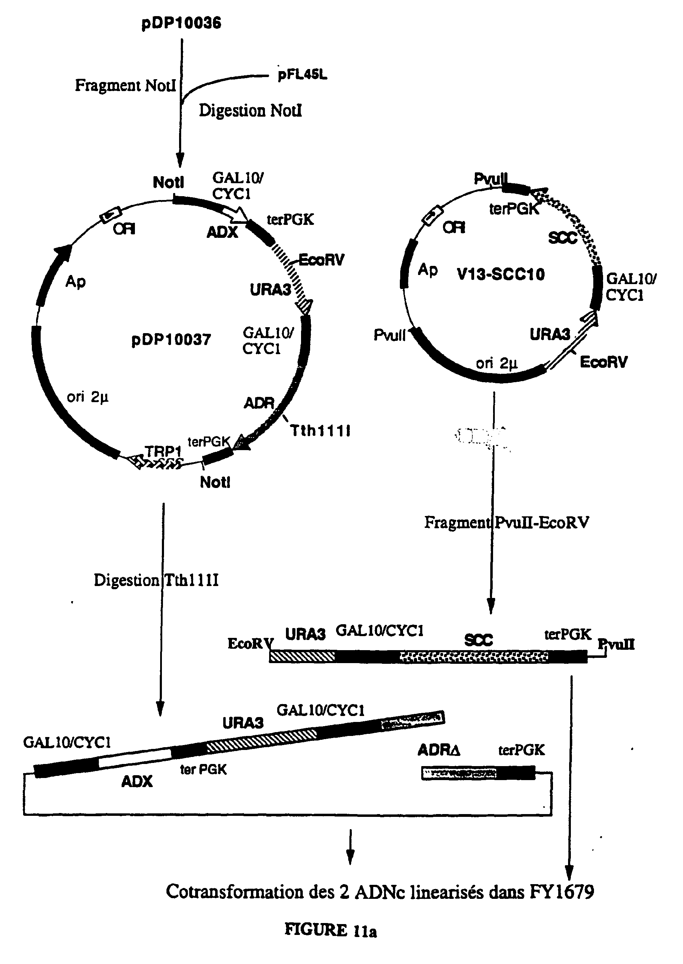

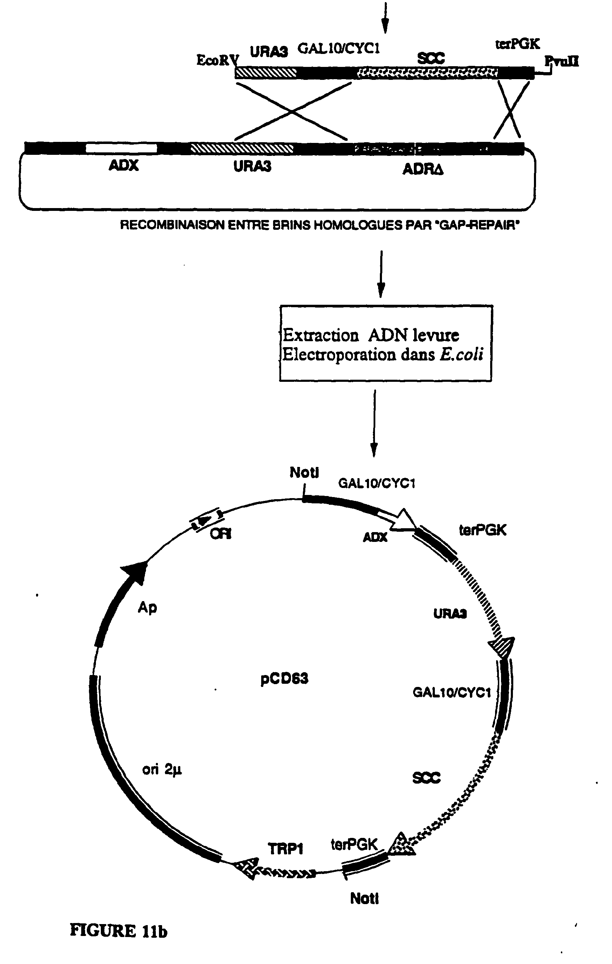

La construction du plasmide pCD63 a été menée comme décrit à la figure 11.

Le fragment NotI de 4961 pb contenant la cassette d'expression ADX, le marqueur de sélection URA3 et la cassette d'expression ADR a été isolé à partir du plasmide pDP10036 préparé ci-dessus et digéré avec l'enzyme de restriction NotI, puis cloné dans le site NotI du site de clonage multiple du plasmide pFL45L (N. Bonneaud et al., Yeast, 7, 609, 1991). Le vecteur ainsi obtenu, dénommé pDP10037 est représenté à la figure 11.

D'une part, le plasmide pDP10037 a été linéarisé par digestion avec l'enzyme Tth111I dont le site de coupure se situe dans le gène codant pour ADRm.

D'autre part, un fragment PvuII-EcoRV de 3476 pb contenant la cassette d'expression P₄₅₀SCC et l'extrémité 5' du marqueur URA3 a été purifié à partir du plasmide V13-SCC10 obtenu précédemment et digéré avec les enzymes de restriction PvuII et EcoRV.

Les deux ADN linéaires respectivement obtenus ont des régions homologues qui correspondent d'une part à l'extrémité 5' du gène URA3 et à GAL10/CYC1, d'autre part à ter PGK comme cela est représenté à la figure 11a.

Ces deux fragments ont été ensuite introduits dans la souche de levure FY1679 (Mata) par co-transformation par la méthode à l'acétate de lithium (D. Gietz et al., déjà cité).

La sélection suivante de recombinants prototrophes pour l'uracile et le tryptophane (URA3⁺, TRP1⁺), a permis d'isoler des clones dans lesquels la cassure double brin générée par digestion par l'enzyme de restriction Tth111I a été réparée par suite de l'intégration de la cassette d'expression P₄₅₀SCC par recombinaison homologue.

La sélection des recombinants (URA3⁺, TRP1⁺) a été réalisée sur le milieu minimum WO enrichi en leucine, histidine et leucine (20 mg/l chacun) mais dépourvu d'uracile et de tryptophane. A partir de 50 clones réunis, l'ADN total a été extrait par la méthode décrite par C. Hoffman et al., (Gene, 57, 267, 1987), puis introduit par électroporation dans la souche d'E. coli XL1-Blue (Stratagène). Les clones transformés par le plasmide généré par "gap repair" ont été sélectionnés sur le milieu riche LB (tryptone 1 %, extrait de levure 0,5 %, NaCl 1 %) contenant 50 mg/l d'ampicilline. A partir d'un des clones sélectionnés, le plasmide, dénommé pCD63, a été extrait selon la méthode décrite par J. Sambrook et al., Molecular Cloning, Cold Spring Harbor Laboratory Press, 1989. Le plasmide pCD63 obtenu contient les cassettes d'expression ADX et P₄₅₀SCC séparées par le marqueur de sélection URA3, comme cela est représenté à la figure 11b.

### b) Transformation de la souche EC01 par le plasmide pCD63

Le plasmide pCD63 a été introduit dans la souche EC01 obtenue ci-dessus par transformation par la méthode à l'acétate de lithium (D. Gietz et al., déjà décrit). La levure transformée a été ensuite cultivée sur le milieu minimum WO décrit précédemment et dépourvu d'uracile, de tryptophane, d'adénine et de leucine mais supplémenté en histidine (20 mg/l).

La souche, dénommée EC01/pCD63, a été ainsi isolée. Un échantillon de la souche, sous la référence EC01/pCD63, a été déposé à la CNCM le 10 février 1995 sous le numéro I-1538**.**

### c) Production in vivo de pregnénolone et de pregnénolone 3-acétate.

La souche EC01/pCD63 a été cultivée à 28°C en aérobiose (130 t/min) dans un Erlenmeyer de 3 litres jusqu'à ce qu'elle atteigne la phase stationnaire de croissance (DO600 = 12 à 13) dans le milieu sélectif SGI décrit à l'exemple 1A et dans lequel la concentration en glucose est de 5 g/l. La culture est ensuite diluée par addition d'un volume du milieu complet YP décrit ci-dessus, puis addition d'éthanol (0,5 % v/v) comme source de carbone. Quand une nouvelle phase stationnaire de croissance a été atteinte (DO600 = 12 à 13), du galactose (20 g/l) est ajouté à la culture, sous forme de solution concentrée (500 g/l), de façon à induire simultanément l'expression des gènes codant pour ADXm, ADRm, P₄₅₀SCC et delta-7 Red qui sont respectivement sous le contrôle du promoteur GAL10/CYC1.

La co-expression des quatre gènes a été mise en évidence par analyse des stérols accumulés respectivement dans les cellules et dans le surnageant de la culture selon le mode opératoire suivant :

Des échantillons de 50 ml de culture sont récoltés après 9 h et 24 h d'induction. Chaque échantillon est centrifugé (4000 g, 10 min, 4°C) de façon à séparer les cellules du milieu de culture.

D'une part, les cellules sont lysées par cassage mécanique en présence de billes de verre selon la méthode indiquée à l'exemple 1F. A partir du lysat ainsi obtenu, les stérols intracellulaires sont ensuite extraits par addition d'un volume d'hexane.

D'autre part, les stérols présents dans le milieu de culture sont extraits directement par addition d'un volume d'hexane.

La composition en stérols extraits est analysée par CPG comme indiqué à l'exemple 1, comparativement à des produits standards.

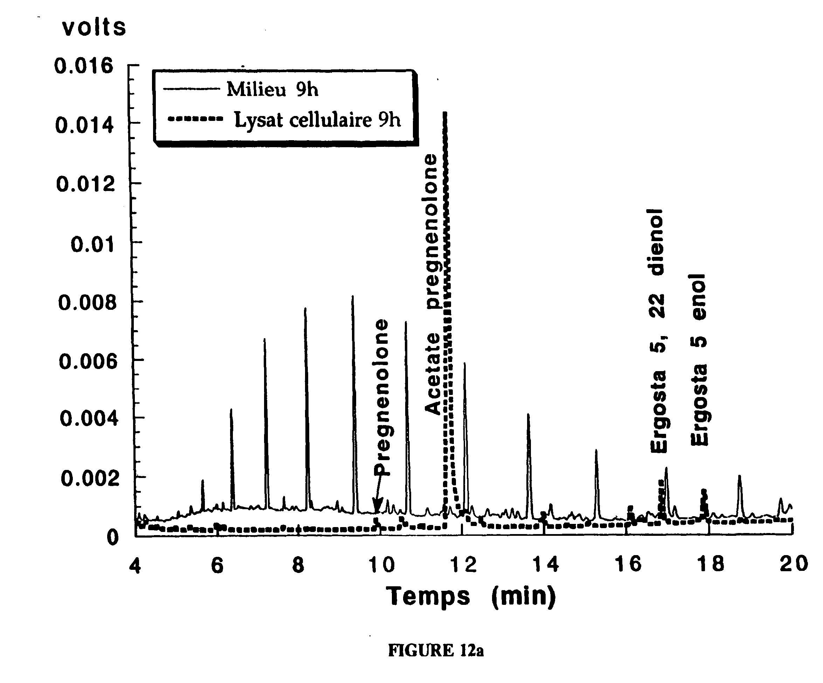

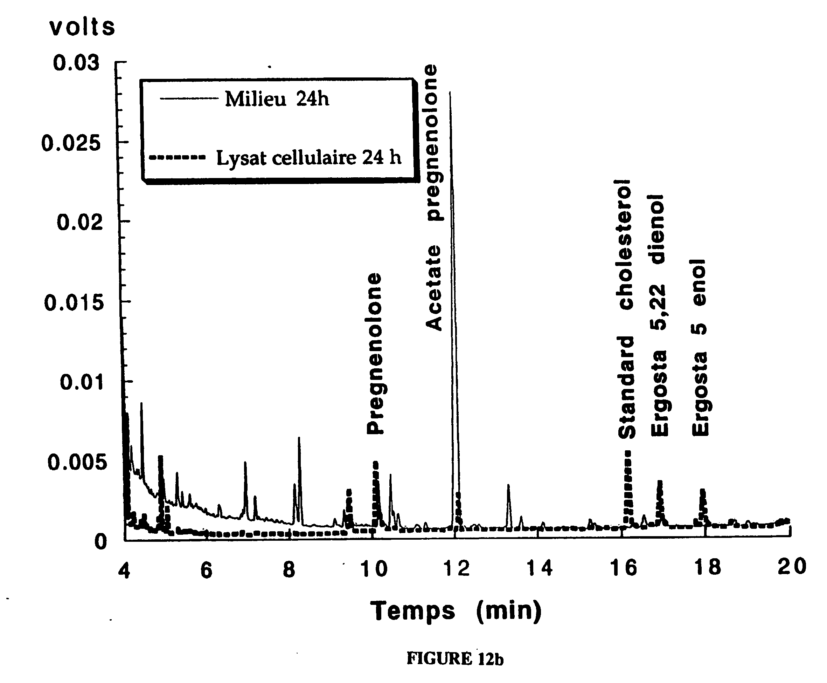

Les résultats obtenus après 9 heures ou après 24 heures d'induction sont montrés respectivement à la figure 12a et à la figure 12b.
Après 9 heures d'induction, la figure 12a montre :
- dans le lysat cellulaire, la présence d'un composé majoritaire ayant un temps de rétention identique à celui de l'acétate de pregnénolone standard (TR = 11,8 mn) alors qu'un pic seulement très faible est présent au temps de rétention de la pregnénolone (TR = 9,9 mn). De faibles quantités de stérols endogènes de la levure réduits en C-7 (ergosta 5-ène 3-ol et ergosta 5,22 diène 3-ol) sont identifiés respectivement à TR = 18 mn et TR = 17 mn. La saponification alcaline du lysat cellulaire avant l'analyse conduit à la présence d'un composé majoritaire co-migrant avec la pregnénolone. Ceci permet de confirmer que le composé accumulé ayant un TR de 11,8 mn correspond à l'acétate de pregnénolone.
- dans le milieu de culture, l'absence significative de pregnénolone ou de son acétate.
Après 24 heures d'induction, la figure 12b montre :
- dans le lysat cellulaire, la présence de faibles quantités de pregnénolone (TR = 10,2 mn) et d'acétate de pregnénolone (TR = 12 mn) ainsi que de stérols endogènes de la levure réduits (TR = 17 mn et TR = 18 mn). Le cholestérol (TR = 16,2 min) est un standard interne ajouté avant l'extraction.
- dans le milieu de culture, la présence majoritaire d'acétate de pregnénolone et d'une quantité mineure de pregnénolone.

Des expériences réalisées en parallèle avec la souche EC01 transformée par un plasmide contrôle tel que pFL45L déjà cité, n'ont montré aucun pic correspondant à la pregnénolone libre ou sous forme acétate.

L'identification des stérols ainsi réalisée montre que la souche de levure EC01/pCD63 a accumulé de la pregnénolone et de l'acétate de pregnénolone en absence de toute source de stérols exogènes, après induction en présence de galactose avec une production majeure après une induction de 9 heures.

Ces résultats démontrent d'une part la mobilisation efficace des stérols endogènes réduits en position C-7 de la souche EC01, d'autre part l'extrême efficacité de couplage de la réaction de coupure de la chaîne latérale des stérols endogènes.

**TABLEAU RECAPITULATIF DES SOUCHES INTEGREES.**

| Souches | Locus d'intégration | Gène intégré | Plasmide transformant | Marqueur plasmidique | Gène(s) sur plasmide | Marqueurs de sélection |
|---|---|---|---|---|---|---|
| CDR01 (Mata) | intergénique LEU2-SPL1 | ADRm | - | - | - | HIS3, URA3, TRP1 |
| ELR01 (Mata) | ADE2 | delta-7Red | - | - | - | ADE2, URA3, LEU2, HIS3, TRP1 |
| EC01 (diploïde) | intergénique LEU2-SPL1 + ADE2 | ADRm delta-7Red | - | - | - | HIS3, URA3, TRP1 |
| EC01/pCD63 | intergénique LEU2-SPL1 + ADE2 | ADRm delta-7RED | pCD63 | URA3 TRP1 | ADXm P450SCC | HIS3 |

### Préparation de l'exemple 4 : construction du plasmide pTG10033

### 1. Dérivé de pUC19 ayant un nouveau site de clonage multiple :

Le vecteur de clonage M13mp19 (C. Yanish-Peron et al., Gene, 33, 103, 1985) a été mutagénisé en utilisant l'oligonucléotide suivant
5' GCGCTCAGCG GCCGCTTTCC AGTCG 3' SEQ ID N° 15
pour introduire un site NotI dans la séquence du gène lacI tronqué et donner le plasmide M13TG724.

Un "polylinker" contenant les sites EcoRI, SnaBI et NotI a été ensuite introduit dans le site EcoRI du plasmide M13TG724 en utilisant les oligonucléotides suivants
5' AATTGCGGCC GCGTACGTAT G 3' SEQ ID N° 16
et
5' AATTCATACG TACGCGGCCG C 3' SEQ ID N° 17
pour donner le plasmide M13TG7244 dans lequel une modification de l'insert a été observée pendant le stade d'amplification. L'insert du plasmide M13TG7244 a la séquence nucléotidique suivante
GAATTCATACGTACGCGGCCGCAATTGCGGCCGGTACGTATAATTCACTGGCCGT
dans laquelle les sites EcoRI, SnaBI et NotI sont soulignés et la séquence lacZ de pUC19 est en italique.
Après digestion du plasmide M13TG7244 par les enzymes de restriction EcoRI et SstI, un "polylinker" contenant les sites MluI et AvrII a été introduit en utilisant les oligonucléotides suivants
5' CAACGCGTCC TAGG 3' SEQ ID N° 18 et
5' AATTCCTAGG ACGCGTTGAG CT 3' SEQ ID N° 19.

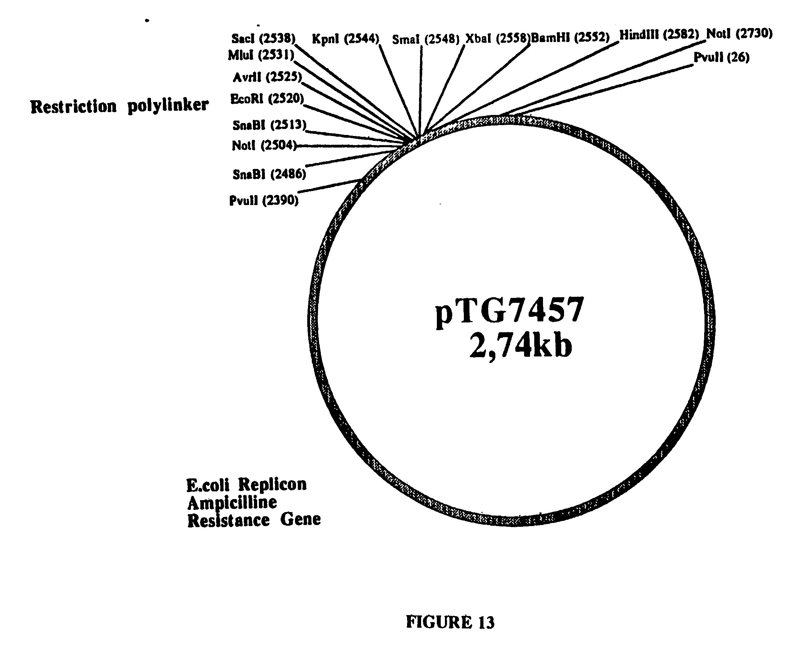

Après digestion avec l'enzyme PvuII, le fragment PwII obtenu a été sous-cloné dans pUC19 (C. Yanish-Perron et al. déjà cité) pour donner le plasmide pTG7457 (figure 13).

### 2. Sous-clonage du terminateur PGK :

pUC19 a été digéré avec les enzymes de restriction BamHI et EcoRI et un nouveau "polylinker" BamHI SstI a été introduit en utilisant les oligonucléotides suivants
5' GATCCGCAGA TATCATCTAG ATCCCGGGTA GAT 3' SEQ ID N° 20,
5' AGAGCTCAAG ATCTACCCGG GATCTAGATG ATATCTGCG 3' SEQ ID N° 21,
5' CTTGAGCTCT ACGCAGCTGG TCGACACCTA GGAG 3' SEQ ID N° 22
et
5' AATTCTCCTA GGTGTCGACC AGCTGCGT 3' SEQ ID N° 23.

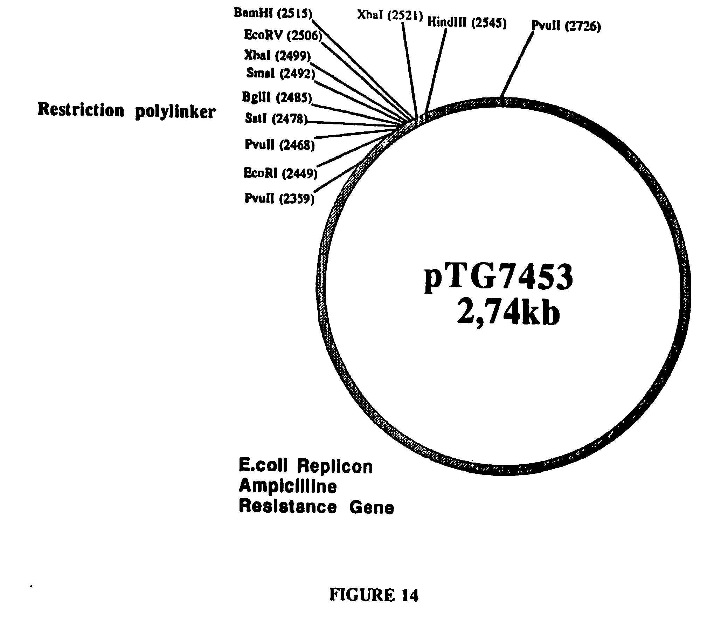

Le plasmide pTG7453 a été ainsi obtenu (figure 14) puis a ensuite été digéré par les enzymes de restriction BamHI et SstI. Les sites du "polylinker" entre BamHI et SstI ont été introduits dans un dérivé du plasmide pTG7457 obtenu ci-dessus et digéré par les enzymes de restriction BamHI et SstI. Le nouveau plasmide obtenu contient les sites PvuII, HindIII, BamHI, EcoRI, XbaI, SmaI, BglII, SstI (=SacI), MluI, AvrII, EcoRI, SnaBI, NotI, SnaBI, PvuI.

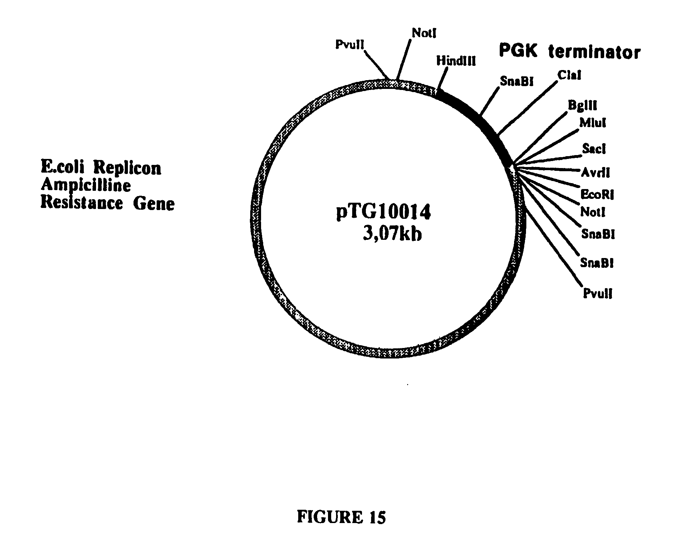

Ce nouveau plasmide a été digéré par les enzymes de restriction BglII et HindIII et un fragment BglII-HindIII contenant le promoteur PGK (R. A. Hitzeman et al., Nucleic Acids Res., 10, 7791, 1982 ; G. Loison et al., Yeast, 5, 497, 1989) a été inséré dans celui-ci pour donner le plasmide pTG10014 (figure 15).

### 3. Sous-clonage de promoteurs :

### a) le promoteur CYC1

Les sites du "polylinker" entre BamHI et SstI du plasmide pTG7453 ont été introduits dans un dérivé du plasmide pTG7457 comme indiqué ci-dessus. Le nouveau plasmide obtenu a été clivé par l'enzyme de restriction SnaBI, puis le fragment RsaI-DraI de 456bp du plasmide pEMBL8 (L. Dente et al., Nucleic Acid Res., 11, 1645, 1983) contenant l'origine de réplication du phage f1 a été introduit pour donner le plasmide pTG7503.

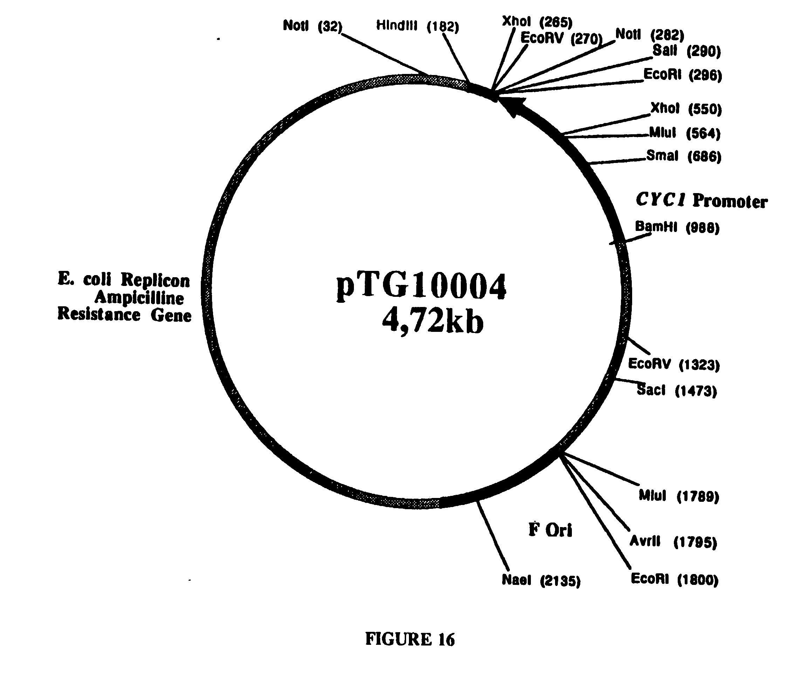

Le fragment BamHI HindIII de 0,78 kb du plasmide pGBSCC-9, préparé dans l'exemple 6 de la demande de brevet européen EP 0340378 et contenant le promoteur CYC1 de S. cerevisiae, un "polylinker" et le terminateur lactase de K. lactis, a été sous-cloné dans le plasmide pTG7503 digéré avec les enzymes de restriction HindIII et BamHI pour donner le plasmide pTG10004 (figure 16).

Les sites XhoI et MluI du promoteur CYC1 ont été ensuite éliminés par mutagénèse dirigée de l'ADN double brin du plasmide pTG10004 en utilisant l'oligonucléotide suivant
5' GCGGATCTGC TCGAAGATTG CCTGCGCGTT GGGCTTGATC 3' SEQ ID N° 24.
Le plasmide pTG10005 ainsi obtenu a ensuite été digéré par les enzymes de restriction SalI et XhoI puis un site MluI a été introduit en utilisant les oligonucléotides suivants
5' TCGACGGACG CGTGG 3' SEQ ID N° 25
et
5' TCGACCACGC GTCC 3' SEQ ID N° 26
pour donner le plasmide pTG10006.

### b) le promoteur GAL10/CYC1

Le plasmide pYeDP1/8-2 (C. Cullin et al., Gene, 203, 1988) a été ouvert avec l'enzyme de restriction XhoI. Les extrémités cohésives créées ont été remplies à l'aide du fragment de Klenow de l'ADN polymérase puis le plasmide a été religaturé. Le plasmide pTG10010 ainsi obtenu dans lequel le promoteur GAL10/CYC1 ne contient plus de site XhoI est utilisé comme matrice pour une amplification par PCR.

### 4. Construction du vecteur d'expression pTG10031

La partie restante de la séquence codante de lacZ a été éliminée dans le plasmide pTG7503 par mutagénèse dirigée en utilisant l'oligonucléotide suivant
5_{'} TGGCCGTCGT TTTACTCCTG CGCCTGATGC GGTAT 3' SEQ ID N° 27
pour donner le plasmide pTG7549.

Le promoteur LacZ présent dans le plasmide pTG7549 a été ensuite délété en utilisant les oligonucléotides suivants
5' GGCCGCAAAA CCAAA 3' SEQ ID N° 28
et
5' AGCTTTTGGT TTTGC 3' SEQ ID N° 29
qui sont insérés dans le plasmide préalablement digéré par les enzymes de restriction NotI et HindIII et qui restaurent les deux sites pour donner le plasmide pTG7553.

Un fragment BamHI MluI contenant le promoteur CYC1 a été obtenu à partir du plasmide pTG10006 digéré par les enzymes de restriction BamHI et MluI et un fragment MluI HindIII contenant le promoteur PGK a été isolé à partir du plasmide pTG10015 digéré par les enzymes de restriction MluI et HindIII. Ces deux fragments ont été ligaturés et le produit de ligature obtenu a été inséré dans le plasmide pTG7553 préalablement digéré par les enzymes de restriction MluI et HindIII.

L'oligonucléotide suivant
5' GATCTATCGA TGCGGCCGCG 3' SEQ ID N° 30
hybridé avec l'oligonucléotide suivant
5' CGCGCGCGGC CGCATCGATA 3' SEQ ID N° 31.
qui constitue un "linker" BamHI MluI contenant des sites ClaI et NotI, a été ajouté et ligaturé pour donner le vecteur d'expression pTG 10031 (figure 17).

Le fragment amplifié par PCR obtenu ci-dessus à partir du plasmide pYeDP1/8-2 a été digéré avec les enzymes de restriction ClaI et SalI puis a été introduit dans le plasmide pTG10031 préalablement digéré avec les mêmes enzymes de restriction pour donner le plasmide pTG10033 (figure 18).

### LISTE DE SEQUENCES

(1) INFORMATIONS GENERALES:
   (i) DEPOSANT:
      (A) NOM: ROUSSEL UCLAF
      (B) RUE: 102, Route de Noisy
      (C) VILLE: ROMAINVILLE
      (E) PAYS: FRANCE
      (F) CODE POSTAL: 93230
      (G) TELEPHONE: 49.91.49.91
      (H) TELECOPIE: 49.91.46.10
   (ii) TITRE DE L' INVENTION: Séquence d'ADN codant pour une proteine d'A. thaliana ayant une activite delta-5,7 sterol,delta-7 reductase, protéine delta-7Red, procède de production, souches de levures transformees, applications.
   (iii) NOMBRE DE SEQUENCES: 31
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (vi) DONNEES DE LA DEMANDE ANTERIEURE:
      (A) NUMERO DE LA DEMANDE: FR 9501723
      (B) DATE DE DEPOT: 15-FEB-1995
   (vi) DONNEES DE LA DEMANDE ANTERIEURE:
      (A) NUMERO DE LA DEMANDE: FR 9506517
      (B) DATE DE DEPOT: 01-JUN-1995
(2) INFORMATIONS POUR LA SEQ ID NO: 1:
   (i) CARACTERISTIQUES DE LA SEQUENCE:
      (A) LONGUEUR: 1496 paires de bases
      (B) TYPE: nucléotide
      (C) NOMBRE DE BRINS: simple
      (D) CONFIGURATION: linéaire
   (ii) TYPE DE MOLECULE: ADNc
   (vi) ORIGINE:
      (A) ORGANISME: Arabidopsis thaliana
   (ix) CARACTERISTIQUE:
      (A) NOM/CLE: CDS
      (B) EMPLACEMENT:76..1365
   (xi) DESCRIPTION DE LA SEQUENCE: SEQ ID NO: 1:
   GTGTGAGTAA TTTAGGTCAA CACAGATCAG AATCTGAGGC TTTGGCCGAG ACGAAGAGAA      60
   AAGCAGAAGA AGAAA ATG GCG GAG ACT GTA CAT TCT CCG ATC GTT ACT TAC      111
                    Met Ala Glu Thr Val His Ser Pro Ile Val Thr Tyr
                     1               5                  10
   GCA TCG ATG TTA TCG CTT CTC GCC TTC TGT CCA CCT TTC GTC ATT CTC      159
   Ala Ser Met Leu Ser Leu Leu Ala Phe Cys Pro Pro Phe Val Ile Leu
            15                  20                  25
   CTA TGG TAC ACA ATG GTT CAT CAG GAT GGT TCT GTT ACT CAG ACC TTT      207
   Leu Trp Tyr Thr Met Val His Gln Asp Gly Ser Val Thr Gln Thr Phe
        30                  35                  40
   GGC TTC TTT TGG GAG AAT GGA GTT CAA GGA CTT ATC AAC ATA TGG CCA      255
   Gly Phe Phe Trp Glu Asn Gly Val Gln Gly Leu Ile Asn Ile Trp Pro
    45                  50                  55                  60
   AGA CCC ACT TTG ATT GCT TGG AAA ATT ATA TTT TGC TAT GGA GCA TTT      303
   Arg Pro Thr Leu Ile Ala Trp Lys Ile Ile Phe Cys Tyr Gly Ala Phe
                    65                  70                  75
   GAA GCT ATT CTT CAG CTG CTT CTG CCT GGT AAA AGA GTT GAG GGT CCA      351
   Glu Ala Ile Leu Gln Leu Leu Leu Pro Gly Lys Arg Val Glu Gly Pro
                80                  85                  90
   ATA TCT CCA GCC GGA AAC CGA CCA GTT TAC AAG GCC AAT GGT CTG GCT      399
   Ile Ser Pro Ala Gly Asn Arg Pro Val Tyr Lys Ala Asn Gly Leu Ala
            95                 100                 105
   GCT TAC TTT GTG ACA CTA GCA ACC CAT CTT GGT CTT TGG TGG TTT GGA      447
   Ala Tyr Phe Val Thr Leu Ala Thr His Leu Gly Leu Trp Trp Phe Gly
       110                 115                 120
   ATC TTC AAC CCT GCA ATT GTC TAT GAT CAC TTG GGT GAA ATA TTT TCG      495
   Ile Phe Asn Pro Ala Ile Val Tyr Asp His Leu Gly Glu Ile Phe Ser
   125                 130                 135                 140
   GCA CTA ATA TTC GGA AGC TTC ATA TTT TGT GTT TTG TTG TAC ATA AAA        543
   Ala Leu Ile Phe Gly Ser Phe Ile Phe Cys Val Leu Leu Tyr Ile Lys
                   145                 150                 155
   GGC CAT GTT GCA CCT TCA TCA AGT GAC TCT GGT TCA TGT GGT AAC CTA        591
   Gly His Val Ala Pro Ser Ser Ser Asp Ser Gly Ser Cys Gly Asn Leu
               160                 165                 170
   ATA ATT GAC TTC TAT TGG GGC ATG GAG TTG TAC CCT CGA ATT GGT AAG        639
   Ile Ile Asp Phe Tyr Trp Gly Met Glu Leu Tyr Pro Arg Ile Gly Lys
           175                 180                 185
   AGC TTT GAC ATC AAG GTG TTT ACT AAT TGC AGA TTC GGA ATG ATG TCT        687
   Ser Phe Asp Ile Lys Val Phe Thr Asn Cys Arg Phe Gly Met Met Ser
       190                 195                 200
   TGG GCA GTT CTT GCA GTC ACG TAC TGC ATA AAA CAG TAT GAA ATA AAT        735
   Trp Ala Val Leu Ala Val Thr Tyr Cys Ile Lys Gln Tyr Glu Ile Asn
   205                 210                 215                 220
   GGC AAA GTA TCT GAT TCA ATG CTG GTG AAC ACC ATC CTG ATG CTG GTG        783
   Gly Lys Val Ser Asp Ser Met Leu Val Asn Thr Ile Leu Met Leu Val
                   225                 230                 235
   TAT GTC ACA AAA TTC TTC TGG TGG GAA GCT GGT TAT TGG AAC ACC ATG        831
   Tyr Val Thr Lys Phe Phe Trp Trp Glu Ala Gly Tyr Trp Asn Thr Met
               240                 245                 250
   GAC ATT GCA CAT GAC CGA GCT GGA TTC TAT ATA TGC TGG GGT TGT CTA        879
   Asp Ile Ala His Asp Arg Ala Gly Phe Tyr Ile Cys Trp Gly Cys Leu
           255                 260                 265
   GTG TGG GTG CCT TCT GTC TAC ACT TCT CCA GGC ATG TAC CTT GTG AAC        927
   Val Trp Val Pro Ser Val Tyr Thr Ser Pro Gly Met Tyr Leu Val Asn
       270                 275                 280
   CAC CCC GTC GAA CTC GGA ACT CAG TTG GCA ATA TAC ATT CTC GTT GCA        975
   His Pro Val Glu Leu Gly Thr Gln Leu Ala Ile Tyr Ile Leu Val Ala
   285                 290                 295                 300
   GGA ATT CTG TGC ATT TAC ATA AAG TAT GAC TGT GAT AGA CAA AGG CAA       1023
   Gly Ile Leu Cys Ile Tyr Ile Lys Tyr Asp Cys Asp Arg Gln Arg Gln
                   305                 310                 315
   GAG TTC AGG AGG ACA AAC GGG AAA TGT TTG GTT TGG GGA AGA GCC CCG       1071
   Glu Phe Arg Arg Thr Asn Gly Lys Cys Leu Val Trp Gly Arg Ala Pro
               320                 325                 330
   TCA AAG ATT GTG GCG TCG TAT ACT ACA ACA TCT GGT GAA ACT AAA ACT       1119
   Ser Lys Ile Val Ala Ser Tyr Thr Thr Thr Ser Gly Glu Thr Lys Thr
           335                 340                 345
   AGT CTT CTC TTA ACG TCT GGA TGG TGG GGA TTG GCT CGT CAT TTC CAT       1167
   Ser Leu Leu Leu Thr Ser Gly Trp Trp Gly Leu Ala Arg His Phe His
       350                 355                 360
   TAT GTT CCT GAG ATC TTA AGT GCT TTC TTC TGG ACC GTA CCG GCT CTC       1215
   Tyr Val Pro Glu Ile Leu Ser Ala Phe Phe Trp Thr Val Pro Ala Leu
   365                 370                 375                 380
   TTC GAT AAC TTC TTG GCA TAC TTC TAC GTC CTC ACC CTT CTT CTC TTT       1263
   Phe Asp Asn Phe Leu Ala Tyr Phe Tyr Val Leu Thr Leu Leu Leu Phe
                   385                 390                 395
   GAT CGA GCC AAG AGA GAC GAT GAC CGA TGC CGA TCA AAG TAT GGG AAA       1311
   Asp Arg Ala Lys Arg Asp Asp Asp Arg Cys Arg Ser Lys Tyr Gly Lys
               400                 405                 410
   TAT TGG AAG CTG TAT TGT GAG AAA GTC AAA TAC AGG ATC ATT CCG GGA       1359
   Tyr Trp Lys Leu Tyr Cys Glu Lys Val Lys Tyr Arg Ile Ile Pro Gly
           415                 420                 425
   ATT TAT TGATTGTAAC GAAGTCTGTT GTTCTCATTT TCTACTTATT ACGTTAATTC        1415
   Ile Tyr
       430
   GAACGTTGGA ATCATCAAAA GACCGAGCCA AAACAAAAAT GCAAATTGAT GCGATAGACA     1475
   TTCTTTTGCT GAAAAAAAAA A                                               1496
(2) INFORMATIONS POUR LA SEQ ID NO: 2:
   (i) CARACTERISTIQUES DE LA SEQUENCE:
      (A) LONGUEUR: 430 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Ala Glu Thr Val His Ser Pro Ile Val Thr Tyr Ala Ser Met Leu
   1 5 10 15
   Ser Leu Leu Ala Phe Cys Pro Pro Phe Val Ile Leu Leu Trp Tyr Thr
   20 25 30
   Met Val His Gln Asp Gly Ser Val Thr Gln Thr Phe Gly Phe Phe Trp
   35 40 45
   Glu Asn Gly Val Gln Gly Leu Ile Asn Ile Trp Pro Arg Pro Thr Leu
   50 55 60
   Ile Ala Trp Lys Ile Ile Phe Cys Tyr Gly Ala Phe Glu Ala Ile Leu
   65 70 75 80
   Gln Leu Leu Leu Pro Gly Lys Arg Val Glu Gly Pro Ile Ser Pro Ala
   85 90 95
   Gly Asn Arg Pro Val Tyr Lys Ala Asn Gly Leu Ala Ala Tyr Phe Val
   100 105 110
   Thr Leu Ala Thr His Leu Gly Leu Trp Trp Phe Gly Ile Phe Asn Pro
   115 120 125
   Ala Ile Val Tyr Asp His Leu Gly Glu Ile Phe Ser Ala Leu Ile Phe
   130 135 140
   Gly Ser Phe Ile Phe Cys Val Leu Leu Tyr Ile Lys Gly His Val Ala
   145 150 155 160
   Pro Ser Ser Ser Asp Ser Gly Ser Cys Gly Asn Leu Ile Ile Asp Phe
   165 170 175
   Tyr Trp Gly Met Glu Leu Tyr Pro Arg Ile Gly Lys Ser Phe Asp Ile
   180 185 190
   Lys Val Phe Thr Asn Cys Arg Phe Gly Met Met Ser Trp Ala Val Leu
   195 200 205
   Ala Val Thr Tyr Cys Ile Lys Gln Tyr Glu Ile Asn Gly Lys Val Ser
   210 215 220
   Asp Ser Met Leu Val Asn Thr Ile Leu Met Leu Val Tyr Val Thr Lys
   225 230 235 240
   Phe Phe Trp Trp Glu Ala Gly Tyr Trp Asn Thr Met Asp Ile Ala His
   245 250 255
   Asp Arg Ala Gly Phe Tyr Ile Cys Trp Gly Cys Leu Val Trp Val Pro
   260 265 270
   Ser Val Tyr Thr Ser Pro Gly Met Tyr Leu Val Asn His Pro Val Glu
   275 280 285
   Leu Gly Thr Gln Leu Ala Ile Tyr Ile Leu Val Ala Gly Ile Leu Cys
   290 295 300
   Ile Tyr Ile Lys Tyr Asp Cys Asp Arg Gln Arg Gln Glu Phe Arg Arg
   305 310 315 320
   Thr Asn Gly Lys Cys Leu Val Trp Gly Arg Ala Pro Ser Lys Ile Val
   325 330 335
   Ala Ser Tyr Thr Thr Thr Ser Gly Glu Thr Lys Thr Ser Leu Leu Leu
   340 345 350
   Thr Ser Gly Trp Trp Gly Leu Ala Arg His Phe His Tyr Val Pro Glu
   355 360 365
   Ile Leu Ser Ala Phe Phe Trp Thr Val Pro Ala Leu Phe Asp Asn Phe
   370 375 380
   Leu Ala Tyr Phe Tyr Val Leu Thr Leu Leu Leu Phe Asp Arg Ala Lys
   385 390 395 400
   Arg Asp Asp Asp Arg Cys Arg Ser Lys Tyr Gly Lys Tyr Trp Lys Leu
   405 410 415
   Tyr Cys Glu Lys Val Lys Tyr Arg Ile Ile Pro Gly Ile Tyr
   420 425 430
(2) INFORMATIONS POUR LA SEQ ID NO: 3:
   (i) CARACTERISTIQUES DE LA SEQUENCE:
      (A) LONGUEUR: 15 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:7
      (D) AUTRES INFORMATIONS:/note= "résidu 7 = Trp ou Tyr"
   (ix) CARACTERISTIQUE:
      (A) NOM/CLE: Peptide
      (B) EMPLACEMENT:12
      (D) AUTRES INFORMATIONS:/note= "résidu 12 = His ou Lys"
   (xi) DESCRIPTION DE LA SEQUENCE: SEQ ID NO: 3:
   Leu Leu Xaa Xaa Gly Trp Xaa Gly Xaa Xaa Arg Xaa Xaa Xaa Tyr
   1               5                   10                  15
(2) INFORMATIONS POUR LA SEQ ID NO: 4:
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:10..29
      (D) AUTRES INFORMATIONS:/note= "SEQUENCE ID No 1 DE 76 A 95"
   (xi) DESCRIPTION DE LA SEQUENCE: SEQ ID NO: 4:
      CGCGGATCCA TGGCGGAGAC TGTACATTC 29
(2) INFORMATIONS POUR LA SEQ ID NO: 5:
   (i) CARACTERISTIQUES DE LA SEQUENCE:
      (A) LONGUEUR: 28 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (10..28)
      (D) AUTRES INFORMATIONS:/note= "SEQUENCE ID No 1 COMPLEMENTAIRE DE 1350 A 1368"
   (xi) DESCRIPTION DE LA SEQUENCE: SEQ ID NO: 5:
      CAGGGTACCT CAATAAATTC CCGGAATG 28
(2) INFORMATIONS POUR LA SEQ ID NO: 6:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..23)
   (xi) DESCRIPTION DE LA SEQUENCE: SEQ ID NO: 6:
      GATTACGCCA AGCTTTTCGA AAC 23
(2) INFORMATIONS POUR LA SEQ ID NO: 7:
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 7:
      AGATCTTGAG AAGATGCGGC CAGCAAAAC 29
(2) INFORMATIONS POUR LA SEQ ID NO: 8:
   (i) CARACTERISTIQUES DE LA SEQUENCE:
      (A) LONGUEUR: 46 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 8:
      GGGGATCCGT GGTCGACGTA ATTTAGTGTG TGTATTTGTG TTTGCG 46
(2) INFORMATIONS POUR LA SEQ ID NO: 9:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..17)
   (xi) DESCRIPTION DE LA SEQUENCE: SEQ ID NO: 9:
      GTAAAACGAC GGCCAGT 17
(2) INFORMATIONS POUR LA SEQ ID NO: 10:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 10:
      TTGAAGGTTC AACATCAATT GATTG 25
(2) INFORMATIONS POUR LA SEQ ID NO: 11:
   (i) CARACTERISTIQUES DE LA SEQUENCE:
      (A) LONGUEUR: 38 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..38)
   (xi) DESCRIPTION DE LA SEQUENCE: SEQ ID NO: 11:
      GTGTGGCGGC CGCCTCCTTG TCAATATTAA TGTTAAAG 38
(2) INFORMATIONS POUR LA SEQ ID NO: 12:
   (i) CARACTERISTIQUES DE LA SEQUENCE:
      (A) LONGUEUR: 34 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 12:
      CAAGGAGGCG GCCGCCACAC AAAAAGTTAG GTGT 34
(2) INFORMATIONS POUR LA SEQ ID NO: 13:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..25)
   (xi) DESCRIPTION DE LA SEQUENCE: SEQ ID NO: 13:
      TCTGCTTCCC TAGAACCTTC TTATG 25
(2) INFORMATIONS POUR LA SEQ ID NO: 14:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..20)
   (xi) DESCRIPTION DE LA SEQUENCE: SEQ ID NO: 14:
      TACATTAGGT CCTTTGTAGC 20
(2) INFORMATIONS POUR LA SEQ ID NO: 15:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 15:
      GCGCTCAGCG GCCGCTTTCC AGTCG 25
(2) INFORMATIONS POUR LA SEQ ID NO: 16:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 16:
      AATTGCGGCC GCGTACGTAT G 21
(2) INFORMATIONS POUR LA SEQ ID NO: 17:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..21)
   (xi) DESCRIPTION DE LA SEQUENCE: SEQ ID NO: 17:
      AATTCATACG TACGCGGCCG C 21
(2) INFORMATIONS POUR LA SEQ ID NO: 18:
   (i) CARACTERISTIQUES DE LA SEQUENCE:
      (A) LONGUEUR: 14 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 18:
      CAACGCGTCC TAGG 14
(2) INFORMATIONS POUR LA SEQ ID NO: 19:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..22)
   (xi) DESCRIPTION DE LA SEQUENCE: SEQ ID NO: 19:
      AATTCCTAGG ACGCGTTGAG CT 22
(2) INFORMATIONS POUR LA SEQ ID NO: 20:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 20:
      GATCCGCAGA TATCATCTAG ATCCCGGGTA GAT 33
(2) INFORMATIONS POUR LA SEQ ID NO: 21:
   (i) CARACTERISTIQUES DE LA SEQUENCE:
      (A) LONGUEUR: 39 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..39)
   (xi) DESCRIPTION DE LA SEQUENCE: SEQ ID NO: 21:
      AGAGCTCAAG ATCTACCCGG GATCTAGATG ATATCTGCG 39
(2) INFORMATIONS POUR LA SEQ ID NO: 22:
   (i) CARACTERISTIQUES DE LA SEQUENCE:
      (A) LONGUEUR: 34 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 22:
      CTTGAGCTCT ACGCAGCTGG TCGACACCTA GGAG 34
(2) INFORMATIONS POUR LA SEQ ID NO: 23:
   (i) CARACTERISTIQUES DE LA SEQUENCE:
      (A) LONGUEUR: 28 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..28)
   (xi) DESCRIPTION DE LA SEQUENCE: SEQ ID NO: 23:
      AATTCTCCTA GGTGTCGACC AGCTGCGT 28
(2) INFORMATIONS POUR LA SEQ ID NO: 24:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 24:
      GCGGATCTGC TCGAAGATTG CCTGCGCGTT GGGCTTGATC 40
(2) INFORMATIONS POUR LA SEQ ID NO: 25:
   (i) CARACTERISTIQUES DE LA SEQUENCE:
      (A) LONGUEUR: 15 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 25:
      TCGACGGACG CGTGG 15
(2) INFORMATIONS POUR LA SEQ ID NO: 26:
   (i) CARACTERISTIQUES DE LA SEQUENCE:
      (A) LONGUEUR: 14 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..14)
   (xi) DESCRIPTION DE LA SEQUENCE: SEQ ID NO: 26:
      TCGACCACGC GTCC 14
(2) INFORMATIONS POUR LA SEQ ID NO: 27:
   (i) CARACTERISTIQUES DE LA SEQUENCE:
      (A) LONGUEUR: 35 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 27:
      TGGCCGTCGT TTTACTCCTG CGCCTGATGC GGTAT 35
(2) INFORMATIONS POUR LA SEQ ID NO: 28:
   (i) CARACTERISTIQUES DE LA SEQUENCE:
      (A) LONGUEUR: 15 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 28:
      GGCCGCAAAA CCAAA 15
(2) INFORMATIONS POUR LA SEQ ID NO: 29:
   (i) CARACTERISTIQUES DE LA SEQUENCE:
      (A) LONGUEUR: 15 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..15)
   (xi) DESCRIPTION DE LA SEQUENCE: SEQ ID NO: 29:
      AGCTTTTGGT TTTGC 15
(2) INFORMATIONS POUR LA SEQ ID NO: 30:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 30:
      GATCTATCGA TGCGGCCGCG 20
(2) INFORMATIONS POUR LA SEQ ID NO: 31:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..20)
   (xi) DESCRIPTION DE LA SEQUENCE: SEQ ID NO: 31:
      CGCGCGCGGC CGCATCGATA 20

## Revendications

1. Séquence d'acide nucléique contenant une séquence codant pour une protéine ayant une activité delta-5,7 stérol, delta-7 réductase, qui présente une identité de séquence d'environ 60% et plus avec la séquence SEQ ID N° 1.

2. Séquence d'acide nucléique selon la revendication 1 dans laquelle l'acide nucléique est un ADN ou un ARN.

3. Séquence d'ADN selon la revendication 1 dans laquelle la séquence codante code pour une protéine d'*A. thaliana* ayant une activité delta-5,7 stérol, delta-7 réductase et ayant la séquence nucléotidique de la séquence SEQ ID N° 1 :

4. Séquence d'ADN codant pour une protéine ayant une activité delta-5,7 stérol, delta-7 réductase et amplifiable par la technique de PCR en utilisant comme amorces des oligonucléotides codant pour une séquence consensus ayant la séquence en acides aminés SEQ ID N° 3 : dans laquelle Xaa en position 7 est Trp ou Tyr et Xaa en position 12 est His ou Lys associés à une amorce oligodT.

5. Protéine d'*A. thaliana* ayant une activité delta-5,7 stérol, delta-7 réductase et ayant la séquence en acides aminés SEQ ID N° 2.

6. Protéine ayant une activité delta-5,7 stérol, delta-7 réductase ayant une séquence en acides aminés présentant une identité de séquence d'environ 60 % et plus avec la séquence SEQ ID N° 2.

7. Protéine ayant une activité delta-5,7 stérol,delta-7 réductase telle qu'obtenue par expression dans une cellule h8te contenant une séquence d'ADN selon l'une quelconque des revendications 1 à 4.

8. Protéine d'*A. thaliana* ayant une activité delta-5,7 stérol, delta-7 réductase telle qu'obtenue par expression dans une cellule h8te contenant une séquence d'ADN codant pour la séquence en acides aminés SEQ ID N° 2.

9. Protéine selon la revendication 7 ou la revendication 8 dans laquelle la cellule hôte est une levure.

10. Anticorps dirigé contre une protéine ayant une activité delta-5,7 stérol,delta-7 réductase selon l'une quelconque des revendications 5 à 9.

11. Vecteur d'expression contenant une séquence d'ADN selon l'une quelconque des revendications 1 à 4.

12. Cellule hôte transformée par un vecteur selon la revendication 11.

13. Procédé de clonage d'un acide nucléique codant pour une protéine ayant une activité delta-5,7 stérol,delta-7 réductase dans un microorganisme comprenant une méthode de criblage choisie parmi :
- la résistance du microorganisme à la nystatine ou à un composé analogue dont la toxicité dépend de la présence de stérols portant une insaturation en position C-7,
- l'hybridation de l'acide nucléique avec la séquence nucléotidique de la séquence SEQ ID N° 1,

14. Cellule hôte transformée selon la revendication 12 dans laquelle l'hôte est une levure ou un champignon filamenteux.

15. Procédé de préparation d'une protéine ayant une activité delta-5,7 stérol, delta-7 réductase dans lequel on cultive une cellule hôte transformée selon l'une quelconque des revendications 12 ou 14 et isole la protéine exprimée.

16. Procédé selon la revendication 15 dans lequel la cellule hôte est une levure transformée dans laquelle la séquence d'ADN codante est placée sous le contrôle d'un promoteur de levure.

17. Procédé de réduction in vitro d'un stérol insaturé en position C-7 dans lequel on incube le stérol à réduire avec la protéine obtenue selon la revendication 15 ou la revendication 16 et on isole éventuellement le stérol réduit obtenu.

18. Procédé de réduction in vivo d'un stérol exogène insaturé en position C-7 dans lequel on incube le stérol avec une cellule hôte transformée selon l'une quelconque des revendications 12 ou 14 et on isole éventuellement le stérol 10 réduit obtenu.

19. Procédé de réduction *in vivo* d'un stérol endogène insaturé en position C-7 dans lequel on cultive une souche hôte transformée selon la revendication 14 et on éventuellement le stérol réduit accumulé.

20. Procédé de réduction in vitro ou in vivo selon l'une quelconque des revendications 17 à 19 dans lequel le stérol réduit obtenu est un substrat de l'enzyme de clivage de la chaîne latérale du cholestérol (P₄₅₀SCC).

21. Procédé de réduction *in vivo* selon la revendication 20 dans lequel le stérol endogène à réduire est l'ergosta 5,7 diène 3-ol, l'ergosta 5,7,24(28) triène 3-ol ou l'ergosta 5,7,22 triène 3-ol ou un mélange de ceux-ci.

22. Procédé de production de pregnénolone dans lequel on cultive une cellule hôte transformée selon la revendication 14, on isole éventuellement le ou les stérols endogènes réduits en position C-7 accumulés, on incube les stérols réduit en présence de P₄₅₀SCC, et éventuellement d'adrénodoxine réductase (ADR) et d'adrénodoxine (ADX) , et on isole éventuellement la pregnénolone obtenue.

23. Procédé selon la revendication 22 dans lequel la cellule hôte est une levure.

24. Cellules de levures transformées exprimant une delta-5, 7 stérol, delta-7 réductase.

25. Souche de levure transformée coexprimant une protéine ayant l'activité delta-5,7 stérol delta-7 réductase, le P₄₅₀SCC, l'ADR et l'ADX et accumulant de la pregnénolone libre ou estérifiée.

26. Souche de levure selon la revendication 25 dans laquelle la protéine ayant l'activité delta-5,7 stérol delta-7 réductase est la protéine d'*A. thaliana* delta-7Red.

27. Souche de levure selon la revendication 26 dénommée BC01/pCD63 déposée à la CNCM le 10 février 1995 sous le numéro I-1538 .

28. Procédé de production de pregnénolone dans lequel on cultive une levure transformée par un ou plusieurs vecteurs permettant la coexpression d'une protéine ayant l'activité delta-5,7 stérol,delta-7 réductase et de P₄₅₀SCC, et éventuellement de l'ADR et de l'ADX, et on isole éventuellement la pregnénolone libre ou estérifiée.

29. Procédé de production de pregnénolone dans lequel on cultive une levure selon la revendication 24.

30. Procédé selon la revendication 29 dans lequel la protéine ayant l' activité delta-5,7 stérol delta-7 réductase est la protéine d'*A. thaliana* delta-7Red.

31. Procédé selon la revendication 30 dans lequel la souche de levure est la souche ECOI/pCD63, déposée à la CNCM le 10 février 1995 sous le numéro I-1538.

32. Méthode de détection de la déficience en delta-5,1 stérol, delta-7 réductase qui comprend l'incubation d'un échantillon contenant de l'ADN génomique humain avec une sonde selon l'une des revendications 1 à 4 dans des conditions d'hybridation standards et la mise en évidence de la fixation ou de l'absence de fixation de la sonde à l'ADN génomique, l'absence de fixation ou la réduction de celle-ci indiquant une déficience congénitale en delta-5,7 stérol,delta-7 réductase.

## Claims

1. Nucleic acid sequence containing a sequence encoding a protein having a delta-5,7 sterol, delta-7 reductase activity, which exhibits a sequence identity of approximately 60% and more with the sequence SEQ ID No. 1.

2. Nucleic acid sequence according to Claim 1, in which the nucleic acid is a DNA or an RNA.

3. DNA sequence according to Claim 1, in which the coding sequence encodes an *A. thaliana* protein having a delta-5,7 sterol, delta-7 reductase activity and having the nucleotide sequence of the sequence SEQ ID No. 1.

4. DNA sequence encoding a protein having a delta-5,7 sterol, delta-7 reductase activity and which can be amplified by the PCR technique using, as primers, oligonucleotides encoding a consensus sequence having the amino acid sequence SEQ ID No. 3; in which Xaa at position 7 is Trp or Tyr and Xaa at position 12 is His or Lys, combined with an oligodT primer.

5. *A. thaliana* protein having a delta-5,7 sterol, delta-7 reductase activity and having the amino acid sequence SEQ ID No. 2.

6. Protein having a delta-5,7 sterol, delta-7 reductase activity and having an amino acid sequence which exhibits a sequence identity of approximately 60% and more with the sequence SEQ ID No.2.

7. Protein having a delta-5,7 sterol, delta-7 reductase activity as obtained by expression in a host cell containing a DNA sequence according to any one of Claims 1 to 4.

8. A. thaliana protein having a delta-5,7 sterol, delta-7 reductase activity as obtained by expression in a host cell containing a DNA sequence encoding the amino acid sequence SEQ ID No. 2.

9. Protein according to Claim 7 or Claim 8, in which the host cell is a yeast.

10. Antibody directed against a protein having a delta-5,7 sterol, delta-7 reductase activity according to any one of Claims 5 to 9.

11. Expression vector containing a DNA sequence according to any one of Claims 1 to 4.

12. Host cell transformed with a vector according to Claim 11.

13. Method for cloning a nucleic acid encoding a protein having a delta-5,7 sterol, delta-7 reductase activity in a microorganism, comprising a screening method selected from:
- the resistance of the microorganism to nystatin or to an analogous compound, the toxicity of which depends on the presence of sterols bearing an unsaturation at position C-7,
- the hybridization of the nucleic acid with the nucleotide sequence of the sequence SEQ ID No.1.

14. Transformed host cell according to Claim 12, in which the host is a yeast or a filamentous fungus.

15. Method for preparing a protein having a delta-5,7 sterol, delta-7 reductase activity, in which a transformed host cell according to either one of Claims 12 and 14 is cultured and the protein expressed is isolated.

16. Method according to Claim 15, in which the host cell is a transformed yeast in which the coding DNA sequence is placed under the control of a yeast promoter.

17. Method for reduction, *in vitro*, of a sterol which is unsaturated at position C-7, in which the sterol to be reduced is incubated with the protein obtained according to Claim 15 or Claim 16, and the reduced sterol obtained is optionally isolated.

18. Method for reduction, *in vivo*, of an exogenous sterol which is unsaturated at position C-7, in which the sterol is incubated with a transformed host cell according to either one of Claims 12 and 14, and the reduced sterol obtained is optionally isolated.

19. Method for reduction, *in vivo*, of an endogenous sterol which is unsaturated at position C-7, in which a transformed host strain according to Claim 14 is cultured, and the accumulated reduced sterol is optionally isolated.

20. Method for reduction, *in vitro,* or *in vivo,* according to any one of Claims 17 to 19, in which the reduced sterol obtained is a substrate for the cholesterol side-chain cleavage enzyme (P₄₅₀SCC).

21. Method for reduction, *in vivo*, according to Claim 20, in which the endogenous sterol to be reduced is ergosta-5,7-dien-3-ol, ergosta-5,7,24(28)-trien-3-ol or ergosta-5,7,22-trien-3-ol, or a mixture thereof.

22. Method for producing pregnenolone, in which a transformed host cell according to Claim 14 is cultured, the accumulated endogenous sterol(s) reduced at position C-7 is (are) optionally isolated, the reduced sterols are incubated in the presence of P₄₅₀SCC and, optionally, of adrenodoxin reductase (ADR) and of adrenodoxin (ADX), and the pregnenolone obtained is optionally isolated.

23. Method according to Claim 22, in which the host cell is a yeast.

24. Transformed yeast cells expressing a delta-5,7 sterol, delta-7 reductase.

25. Transformed yeast strain which coexpresses a protein having the delta-5,7 sterol, delta-7 reductase activity, P₄₅₀SCC, ADR and ADX and which accumulates free or esterified pregnenolone.

26. Yeast strain according to Claim 25, in which the protein having the delta-5,7 sterol, delta-7 reductase activity is the *A. thaliana* protein delta-7Red.

27. Yeast strain according to Claim 26, called EC01/pCD63, deposited with the CNCM [French national microorganism culture collection] on 10 February 1995 under number I-1538.

28. Method for producing pregnenolone, in which a yeast transformed with one or more vectors enabling the coexpression of a protein having the delta-5,7 sterol, delta-7 reductase activity and of P₄₅₀SCC and, optionally, of ADR and of ADX is cultured, and the free or esterified pregnenolone is optionally isolated.

29. Method for producing pregnenolone, in which a yeast according to Claim 24 is cultured.

30. Method according to Claim 29, in which the protein having the delta-5,7 sterol, delta-7 reductase activity is the *A. thaliana* protein delta-7Red.

31. Method according to Claim 30, in which the yeast strain is the EC01/pCD63 strain deposited with the CNCM on 10 February 1995 under number 1-1538.

32. Method for detecting delta-5,7 sterol, delta-7 reductase deficiency, which comprises incubating a sample containing human genomic DNA with a probe according to one of Claims 1 to 4, under standard hybridization conditions, and demonstrating the binding or the absence of binding of the probe to the genomic DNA, an absence of binding or a reduction in said binding indicating a congenital delta-5,7 sterol, delta-7 reductase deficiency.

## Patentansprüche

1. Nukleinsäuresequenz, enthaltend eine Sequenz, die für ein Protein mit einer delta-5,7-Sterol-delta-7-Reduktase-Aktivität codiert und die eine Sequenzidentität von etwa 60% und mehr mit der Sequenz SEQ ID NR: 1 aufweist.

2. Nukleinsäuresequenz nach Anspruch 1, wobei die Nukleinsäure eine DNA oder eine RNA ist.

3. DNA-Sequenz nach Anspruch 1, wobei die codierende Sequenz für ein Protein von *A. thaliana* mit einer delta-5,7-Sterol-delta-7-Reduktase-Aktivität codiert sowie die Nukleotidsequenz der SEQ ID NR: 1 aufweist.

4. DNA-Sequenz, die für ein Protein mit einer delta-5,7-Sterol-delta-7-Reduktase-Aktivität codiert und durch die PCR-Technik amplifizierbar ist, wobei man als Primer Oligonukleotide verwendet, die für eine Konsensussequenz mit der Aminosäuresequenz SEQ ID NR: 3: codieren, wobei Xaa an Position 7 für Trp oder Tyr steht und Xaa an Position 12 für His oder Lys steht, und die mit einem OligodT-Primer verbunden sind.

5. Protein von *A. thaliana* mit einer delta-5,7-Sterol-delta-7-Reduktase-Aktivität und mit der Aminosäuresequenz SEQ ID NR: 2.

6. Protein mit einer delta-5,7-Sterol-delta-7-Reduktase-Aktivität und einer Aminosäuresequenz, die eine Sequenzidentität von etwa 60% und mehr mit der SEQ ID NR: 2 aufweist.

7. Protein mit einer delta-5,7-Sterol-delta-7-Reduktase-Taktivität, das durch Expression in einer Wirtszelle, die eine DNA-Sequenz nach einem der Ansprüche 1 bis 4 enthält, erhalten wird.

8. Protein von *A. thaliana* mit einer delta-5,7-Sterol-delta-7-Reduktase-Aktivität, das erhalten wird durch Expression in einer Wirtszelle, die eine DNA-Sequenz, die für die Aminosäuresequenz SEQ ID NR: 2 codiert, enthält.

9. Protein nach Anspruch 7 oder Anspruch 8, wobei die Wirtszelle eine Hefe ist.

10. Antikörper, der gegen ein Protein mit einer delta-5,7-Sterol-delta-7-Reduktase-Aktivität nach einem der Ansprüche 5 bis 9 gerichtet ist.

11. Expressionsvektor, der eine DNA-Sequenz nach einem der Ansprüche 1 bis 4 enthält.

12. Wirtszelle, die mit einem Vektor nach Anspruch 11 transformiert ist.

13. Verfahren zur Klonierung eine Nukleinsäure, die für ein Protein mit einer delta-5,7-Sterol-delta-7-Reduktase-Aktivität codiert, in einen Mikroorganismus, umfassend ein Screening-Verfahren, das aus Folgenden ausgewählt ist:
- Resistenz des Mikroorganismus gegen Nystatin oder eine analoge Verbindung, deren Toxizität von der Anwesenheit von Sterolen mit einer Ungesättigtheit an Position C-7 abhängt,
- Hybridisierung der Nukleinsäure mit der Nukleotidsequenz SEQ ID NR: 1.

14. Transformierte Wirtszelle nach Anspruch 12, wobei der Wirt eine Hefe oder ein Fadenpilz ist.

15. Verfahren zur Herstellung eines Proteins mit einer delta-5,7-Sterol-delta-7-Reduktase-Aktivität, wobei man eine transformierte Wirtszelle nach einem der Ansprüche 12 oder 14 kultiviert und das exprimierte Protein isoliert.

16. Verfahren nach Anspruch 15, wobei die Wirtszelle eine transformierte Hefezelle ist, in der die codierende DNA-Sequenz unter die Kontrolle eines Hefepromotors gestellt wurde.

17. Verfahren zur Tn-vitxo-Reduktion eines an Position C-7 ungesättigten Sterols, wobei man das zu reduzierende Sterol mit dem gemäß Anspruch 15 oder Anspruch 16 erhaltenen Protein inkubiert und gegebenenfalls das erhaltene reduzierte Sterol isoliert.

18. Verfahren zur In-vivo-Reduktion eines exogenen, an Position C-7 ungesättigten Sterols, wobei man das Sterol mit einer transformierten Wirtszelle nach einem der Ansprüche 12 oder 14 inkubiert und gegebenenfalls das erhaltene reduzierte Sterol isoliert.

19. Verfahren zur In-vivo-Reduktion eines endogenen, an Position C-7 ungesättigten Sterols, wobei man einen transformierten Wirtsstamm nach Anspruch 14 kultiviert und gegebenenfalls das akkumulierte reduzierte Sterol isoliert.

20. Verfahren zur *In-vitro*- oder *In-vivo*-Reduktion nach einem der Ansprüche 17 bis 19, wobei das erhaltene reduzierte Sterol ein Substrat für ein Sterol-Seitenketten-spaltendes Enzym (P₄₅₀SCC) ist.

21. Verfahren zur In-vivo-Reduktion nach Anspruch 20, wobei das zu reduzierende endogene Sterol Ergosta-5,7-dien-3-ol, Ergosta-5,7,24(28)-trien-3-ol oder Ergosta-5,7,22-trien-3-ol oder ein Gemisch von diesen ist.

22. Verfahren zur Herstellung von Pregnolon, wobei man eine transformierte Wirtszelle nach Anspruch 14 kultiviert, gegebenenfalls das oder die akkumulierte(n) endogene(n), an Position C-7 reduzierte(n) Sterol(e) isoliert, die reduzierten Sterole in Gegenwart von P₄₅₀SCC und gegebenenfalls Adrenodoxin-Reduktase (ADR) und Adrenodoxin (ADX) inkubiert und gegebenenfalls das erhaltene Pregnolon isoliert.

23. Verfahren nach Anspruch 22, wobei die Wirtszelle eine Hefe ist.

24. Transformierte Hefezellen, die eine delta-5,7-Sterol-delta-7-Reduktase exprimieren.

25. Transformierter Hefestamm, der ein Protein mit delta-5,7-Sterol-delta-7-Reduktase-Aktivität, P₄₅₀SCC, ADR und ADX coexprimiert und freies oder verestertes Pregnolon akkumuliert.

26. Hefestamm nach Anspruch 25, wobei es sich bei dem Protein mit delta-5,7-Sterol-delta-7-Reduktase-Aktivität um das delta-7-Red-Protein von *A. thaliana* handelt.

27. Hefestamm nach Anspruch 26, der als EC01/pCD63 bezeichnet wird und bei der CNCM am 10. Februar 1995 unter der Nummer I-1538 hinterlegt wurde.

28. Verfahren zur Herstellung von Pregnolon, wobei man eine Hefe kultiviert, die mit einem oder mehreren Vektoren transformiert ist, welche(r) die Coexpression eines Proteins mit delta-5,7-Sterol-delta-7-Reduktase-Aktivität und von P₄₅₀SCC und gegebenenfalls ADR und ADX gestatte(t/n), und gegebenenfalls das freie oder veresterte Pregnolon isoliert.

29. Verfahren zur Herstellung von Pregnolon, wobei man eine Hefe nach Anspruch 24 kultiviert.

30. Verfahren nach Anspruch 29, wobei es sich bei dem Protein mit delta-5,7-Sterol-delta-7-Reduktase-Aktivität um das delta-7-Red-Protein von *A. thaliana* handelt.

31. Verfahren nach Anspruch 30, wobei der Hefestamm der Stamm EC01/pCD63 ist, der bei der CNCM am 10. Februar 1995 unter der Nummer 1-1538 hinterlegt wurde.

32. Verfahren zum Nachweis eines Mangels an delta-5,7-Sterol-delta-7-Reduktase, bei dem man eine Probe, die menschliche genomische DNA enthält, mit einer Sonde nach einem der Ansprüche 1 bis 4 unter Standard-Hybridisierungsbedingungen inkubiert und die Bindung oder das Fehlen von Bindung der Sonde an die genomische DNA nachweist, wobei das Fehlen von Bindung oder eine verringerte Bindung auf einen congenitalen Mangel an delta-5,7-Sterol-delta-7-Reduktase hinweisen.
